# FASCICULE DE BREVET EUROPEEN

(11) **EP 3 484 891 B1**
(45) Date de publication et mention de la délivrance du brevet: **14.04.2021**
(21) Numéro de dépôt: 17748542.2
(22) Date de dépôt: 13.07.2017
(51) Int. Cl.: C07D 498/04, C12Q 1/68, C12N 9/99, C12N 9/12

(54) **RÉACTIFS POUR LA PROTECTION RÉVERSIBLE DE MOLÉCULES BIOLOGIQUES**
REAGENZIEN ZUM REVERSIBLEN SCHUTZ BIOLOGISCHER MOLEKÜLE
REAGENTS FOR REVERSIBLY PROTECTING BIOLOGICAL MOLECULES

(30) Priorité: 13.07.2016 FR 1656791
(43) Date de publication de la demande: 22.05.2019
(73) Titulaire: Biomérieux, 69280 Marcy-L'Etoile (FR); Université de Caen Normandie, 14032 Caen Cedex 5 (FR)
(72) Inventeur: URSUEGUI, Sylvain, 75013 Paris (FR); LAURENT, Alain, 38100 Grenoble (FR); LAAYOUN, Ali, 38260 La Frette (FR); FABIS, Frédéric, 14000 Caen (FR)
(74) Mandataire: Plasseraud IP
(86) Numéro de dépôt international: PCT/FR2017/051925
(87) Numéro de publication internationale: WO 2018/011527

(56) Documents cités:
- EP-A1- 0 771 870
- WO-A1-2008/149168
- WO-A1-2014/019966
- WO-A2-2004/052312
- ISIDOR MORRIS HEILBRON ET AL: "CCXCII.-Chemical reactivity and conjugation. Part II. The reactivity of the 2-methyl group in the 4-quinazolone series", JOURNAL OF THE CHEMICAL SOCIETY, TRANSACTIONS, vol. 127, 1925, pages 2167-2175, XP055327693, ISSN: 0368-1645, DOI: 10.1039/CT9252702167
- TATSUO NAGASAKA ET AL: "Stereoselective synthesis of tilivalline", THE JOURNAL OF ORGANIC CHEMISTRY, vol. 63, no. 20, 1998, pages 6797-6801, XP002136000, ISSN: 0022-3263, DOI: 10.1021/JO972158G
- G. NORCINI ET AL: "Synthesis and pharmacological evaluation of tyramine congeners containing fused heterocyclic rings", EUROPEAN JOURNAL OF MEDICINAL CHEMISTRY, vol. 28, 1993, pages 505-511, XP023870315, ISSN: 0223-5234, DOI: 10.1016/0223-5234(93)90018-A [extrait le 1993-01-01]

## Description

La présente invention concerne des réactifs pour la protection réversible des molécules biologiques. Elle porte en particulier sur des composés dérivés d'anhydride aza-isatoïque et leurs utilisations pour la protection de molécules biologiques, notamment les enzymes en vue de bloquer leur activité. L'invention vise également les molécules biologiques ainsi protégées et les procédés de mise en œuvre de ces réactifs.

### ETAT DE LA TECHNIQUE

Les tests modernes de diagnostic in-vitro, utilisant des techniques de biologie moléculaire en vue de la détection spécifique, rapide et quantitative d'acides nucléiques cibles présents dans un échantillon biologique utilisent le plus souvent des enzymes polymérases permettant l'amplification d'acides nucléiques cibles à partir d'amorces spécifiques.

Des polymérases dites « hot-start » ont été développées car elles présentent de nombreux avantages par rapport à leur version native et donnent en particulier de meilleures performances de sensibilité aux tests de diagnostic.

Le principe de fonctionnement d'une polymérase « hot-start » est de bloquer son activité de façon transitoire soit par un anticorps, un aptamère ou par un agent chimique puis de restaurer l'activité dès l'élévation de température pendant l'amplification PCR.

Le blocage par un agent chimique est tout particulièrement avantageux en raison de son coût peu élevé. L'inactivation des polymérases par une acylation des fonctions amine des résidus lysine a été décrite dans l'état de la technique (voir par exemple : Enzyme and Microbial Technology 36 (2005) 947-952 « Thermally reversible inactivation of Taq polymerase in an organic solvent for application in hot start PCR » Ariel Louwrier, Anne van der Valk). Lors de l'étape de dénaturation à 95°C pendant le premier cycle de PCR ou pendant une étape de préactivation à température élevée, les groupes protecteurs sont clivés et l'activité de l'enzyme est restaurée.

Les brevets US5677152 et US5773258 décrivent l'utilisation d'anhydride citraconique et ses dérivés en milieu aqueux pour protéger temporairement les groupements NH₂ des lysines de la *Taq* polymérase. L'amide générée après réaction de l'anhydride et d'une amine est ensuite hydrolysable par le milieu rendu acide après traitement thermique en présence de tampon Tris utilisés dans les réactions PCR.

Il a été par ailleurs démontré que les dérivés d'anhydride isatoïque sont capables de réagir avec des groupements nucléophiles tels que les amines présentes sur les protéines. En 1982, Moorman A.R. et al. ont décrit l'acylation d'une protéine, la chymotripsine α, par l'anhydride isatoïque (Moorman A.R., Abeles R.H. J. Am. Chem. Soc., 1982, 104, 6785-6786). Cette réaction entraîne l'inactivation de la protéine en générant des dérivés anthraniliques stables. Plus récemment, Hooker et al. ont utilisé la réactivité de l'anhydride isatoïque pour modifier les résidus lysines de molécules de lysozyme et ainsi introduire des motifs anilines qui peuvent ensuite être fonctionnalisés via un couplage oxydant (Hooker J. M., Esser-Kahn A. B., B. Francis M. B. J. Am. Chem. Soc., 2006, 106, 1558-1559). L'acylation du groupement amine présent sur la chaîne latérale des lysines par l'anhydride isatoïque conduit ainsi à la formation de liaisons amides très stables.

La demande de brevet FR1257526 décrit par ailleurs des agents acylants, dérivés d'anhydride isatoïques pour la fonctionnalisation, le marquage, la capture ou la séparation d'acide ribonucléiques (ARN) ou d'acide nucléiques chimériques (ARN/ADN). Les agents acylants sont plus spécifiquement décrits en vue de la fixation de groupement d'intérêt sur ces molécules biologiques.

La présente invention se propose de fournir de nouveaux réactifs pour la protection réversible de molécules biologiques, et notamment pour la préparation d'enzymes dites « Hot Start », c'est-à-dire qui peuvent être aisément déprotégées par un traitement thermique, lesdits nouveaux réactifs présentant de préférence un ou plusieurs des avantages suivants :
- ils sont bon marchés,
- ils sont stables,
- ils sont solubles en milieu aqueux,
- ils sont efficaces quant à leur vitesse de réaction, mais également quant à leur vitesse de déprotection,
- le produit de leur réaction avec une biomolecule est stable,
- ils ne nécessitent pas l'utilisation de tampon Tris pour la déprotection à chaud et/ou,
- ils ne génèrent pas de réactions secondaires indésirables.

Les réactifs décrits dans la présente demande sont ainsi particulièrement appropriés pour inactiver transitoirement des enzymes impliquées dans la polymérisation des acides nucléiques comme la *Taq* polymérase ou certaines transcriptase inverses, et plus généralement des enzymes utilisées dans les techniques de diagnostic in vitro.

### RESUME

Ainsi, la présente invention a pour objet un composé de formule (I) suivante: 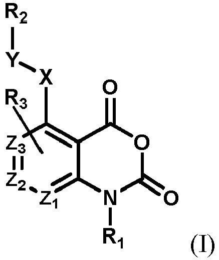 dans laquelle
X est une liaison covalente ou un alkyle en C₁ à C₄,
Y est un radical nucléophile choisi parmi O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, OC(O)-NH-O, NH-C(O)-NH-O, O-C(O)-O-NR₄, NH-C(O)-O-NR₄, C(O)-S, de préférence O, S, NR₄, O-NR₄, NH-O, ou NH-NR₄, et R₄ est H ou un groupe alkyle en C₁ à C₄
et lorsque Y est choisi parmi O, S et NR₄, X n'est pas une liaison,
Z₁, Z₂, Z₃ représentent chacun indépendamment l'un de l'autre, N ou C, de préférence Z₃ représente C, plus préférentiellement Z₃ est C et R₃ est en position Z₃,
R₁ est H, un groupe alkyle en C₁ à C₆, substitué ou non, un alcényle substitué ou non, un groupe aryle substitué ou non, ou un hétérocycle substitué ou non, de préférence R₁ est un groupe méthyle ou éthyle.
R₂ est un groupement protecteur thermolabile et/ou acidolabile,
R₃ est H, un groupe alkyle en C₁ à C₁₂ substitué ou non, par exemple, un groupe *iso-*propyle, isobutyle, sec-butyle, *tert*-butyle, isopentyle ou 2,2-diméthylpropyle, un groupe aryle substitué ou non, un hétérocycle substitué ou non, un groupe acyle, un groupe alcényle substitué ou non, un halogène (e.g. F, Cl, Br et I), ou un groupe cyano.

Dans un mode de réalisation préféré, le composé selon l'invention est de formule (II) suivante :

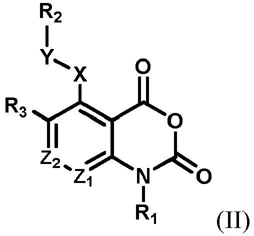

Dans laquelle X, Y, Z₁, Z₂, R₁, R₂ et R₃ sont tels que définis plus haut pour la formule (I).

Dans un mode de réalisation spécifique, le groupement thermolabile et/ou acidolabile R₂ dans les formules (I) et (II) ci-dessus est choisi parmi les groupements *tert*-butoxycarbonyl (BOC), phénoxyacétyle substitué ou non, trityle, méthoxytrityle, diméthoxytrityle ou citraconyle.

Dans un autre mode de réalisation particulier, qui peut être combiné avec les précédents modes de réalisation, R₁ est un groupe méthyle.

Dans un autre mode de réalisation particulier, éventuellement combiné avec les précédents modes de réalisation, R₃ est l'iode.

Dans un autre mode de réalisation particulier, qui peut être combiné avec les précédents modes de réalisation, Z₁ est N et Z₂ est C.

En particulier, l'invention porte sur l'un des composés de structures suivantes :

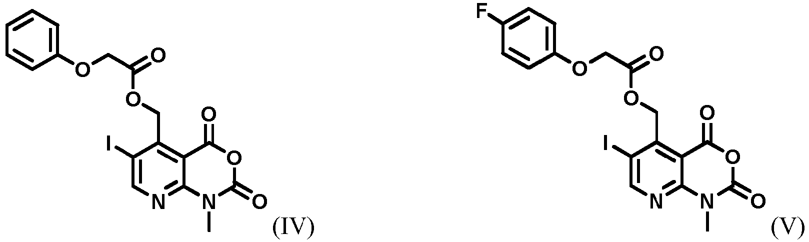

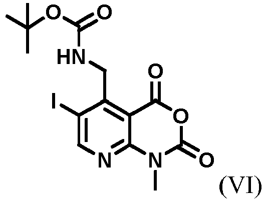

L'invention vise également un procédé de préparation d'une molécule biologique protégée, comprenant la mise en contact d'un composé selon l'invention tel que décrit ci-dessus avec une molécule biologique comprenant un ou plusieurs groupes nucléophiles dans des conditions permettant l'acylation d'un ou plusieurs groupes nucléophiles de ladite molécule biologique, pour former une molécule biologique protégée.

Dans un mode de réalisation plus particulièrement préféré, la molécule biologique comprend des fonctions amines. En particulier, la molécule biologique est une protéine qui comprend des fonctions nucléophiles et notamment les fonctions amines de ses résidus lysines ou de l'acide aminé terminal, les fonctions alcools des sérines, et/ou les fonctions thiols des cystéines.

L'invention porte en particulier sur une molécule biologique protégée, et représentée par la formule (III) suivante : 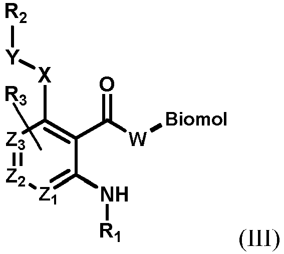 dans laquelle
Biomol est une molécule biologique ;
W est un radical nucléophile de la molécule biologique, de préférence NH, S ou O; et,
X est une liaison covalente ou un alkyle en C₁ à C₄,
Y est un radical nucléophile choisi parmi O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-NH-O, O-C(O)-O-NR₄, NH-C(O)-O-NR₄, C(O)-S, de préférence O, S, NR₄, O-NR₄, NH-O, ou NH-NR₄, et R₄ est H ou un groupe alkyle en C₁ à C₄
et lorsque Y est choisi parmi O, S et NR₄, X n'est pas une liaison,
Z₁, Z₂, Z₃ représentent chacun indépendamment l'un de l'autre, N ou C, de préférence Z₃ représente C, plus préférentiellement Z₃ est C et R₃ est en position Z₃,
R₁ est H, un groupe alkyle en C₁ à C₆, substitué ou non, un alcényle substitué ou non, un groupe aryle substitué ou non, ou un hétérocycle substitué ou non, de préférence R₁ est un groupe méthyle ou éthyle,
R₂ est un groupement protecteur thermolabile et/ou acidolabile,
R₃ est H, un groupe alkyle en C₁ à C₁₂ substitué ou non, par exemple un groupe iso-propyl, isobutyl, sec-butyl, tert-butyl, isopentyl ou 2,2-diméthylpropyl, un groupe aryle substitué ou non, un hétérocycle substitué ou non, un groupe acyle, un groupe alcényle substitué ou non, un halogène (e.g. F, Cl, Br et I), ou un groupe cyano.

Dans un mode de réalisation spécifique, Biomol est choisie parmi les protéines et notamment, les enzymes.

Dans un mode de réalisation plus particulièrement préféré, Biomol est une enzyme destinée à être utilisée dans une réaction de polymérisation d'acide nucléique, par exemple une ADN polymérase.

Sont également décrits des procédés de déprotection des groupes nucléophiles d'une molécule biologique protégée par les composés selon l'invention, ledit procédé comprenant une étape de clivage du ou des groupements thermolabiles et/ou acidolabiles R₂, par traitement thermique et/ou acide respectivement et la déprotection concomitante des groupes nucléophiles de la molécule biologique.

Ainsi, l'invention concerne également les utilisations d'un composé selon l'invention pour l'inactivation réversible d'une enzyme. Dans un mode préféré, le composé selon l'invention est utilisé pour l'inactivation réversible d'une enzyme destinée à être utilisée dans une réaction de polymérisation d'acide nucléique, par exemple une ADN polymérase. L'invention vise en outre un procédé d'amplification d'un acide nucléique comprenant la mise en œuvre d'une enzyme polymérase inactivée par les composés selon l'invention, et au moins une étape de traitement thermique à une température permettant le clivage du ou des groupements thermolabiles R₂ et la déprotection des groupes nucléophiles, ladite étape de traitement thermique précédant ladite étape d'amplification d'acide nucléique.

### Définitions

Le terme « substitué ou non » signifie qu'un ou plusieurs hydrogène(s) présent(s) dans un groupe peut être substitué par un groupe fonctionnel choisi parmi les groupes amine, imine, nitrile, cyano, amide, imide, hydroxyle, alcoxyle, carbonyle, carboxyle, ester, thiol, thioéther, thioester et halogénure.

Le terme « un groupe alkyle en Cx à Cy » désigne une chaîne alkyle linéaire ou ramifiée, ou un cycloalkyle, ayant x à y atomes de carbone. Comme exemple de chaîne alkyle linéaire on peut citer: méthyle, éthyle, n-propyle, n-butyle, n-pentyle, n-hexyle, n-heptyle, n-octyle, n-nonyle et n-décyle. Comme exemple de chaîne alkyle ramifiée, on peut citer: iso-propyle, isobutyle, sec-butyle et tert-butyle, isopentyle, 2,2-diméthylpropyle, iso-octyle, iso-nonyle et iso-décyle. Comme exemple de cycloalkyle, on peut citer cyclopentyle, cyclohexyle, cycloheptyle et cyclooctyle.

Le terme « aryle » désigne des hydrocarbures cycliques aromatiques ne comprenant pas d'hétéroatomes dans le cycle. Par exemple, le groupe aryle peut comprendre entre 6 et 14 atomes de carbones dans la partie cycle aromatique. Comme exemple de group aryle, on peut citer : phényle, biphényle, phénanthrényle, pyrényle, chrysényle, anthracényle et naphtyle.

Le terme « hétérocycle » désigne un groupe comprenant au moins un cycle, saturé ou insaturé, dont au moins un des atomes du cycle est un hétéroatome, comme par exemple N, O ou S. Un hétérocycle peut être composé de plusieurs cycles condensés. Par exemple, l'hétérocycle peut comprendre entre 6 et 14 atomes dans la partie cyclique.

Le terme « acyle » désigne un groupe comprenant un groupe carbonyle, le groupe acyle étant lié à la molécule via l'atome de carbone du carbonyle. Cet atome de carbone est également lié à un autre atome de carbone appartenant à un groupe alkyle substitué ou non, un groupe aryle substitué ou non ou un hétérocycle substitué ou non. Par exemple, le groupe acyle comprend entre 2 et 12 atomes de carbone.

Le terme « alcényle » désigne une chaîne alkyle linéaire ou ramifiée, ou un cycloalkyle, comprenant au moins une double liaison carbone-carbone. Comme exemple de groupes alcényle, on peut citer : vinyle, -CH=CH(CH₃), -CH=C(CH₃)₂, -C(CH₃)=CH₂, - C(CH₃)=CH(CH₃), -C(CH₂CH₃)=CH₂, cyclohexényle, cyclopentényle, cyclohexadiényle, butadiényle, pentadiényle, et hexadiényle. Par exemple, le groupe alcényle comprend entre 2 et 12 atomes de carbone.

Le terme « nucléophile » ou « groupe nucléophilique » désigne un groupe capable de former une liaison covalente avec un site réactif (électrophile) en donnant les deux électrons nécessaires à la création de la liaison, dans des conditions de réaction appropriées. Un ou plusieurs groupes nucléophiles peuvent être naturellement présents sur une molécule biologique, par exemple le groupe amine d'une lysine, l'amine terminale d'une chaine polypeptidique, l'alcool d'une sérine ou encore le groupe thiol d'une cystéine présente sur une protéine ou une enzyme.

Le terme « groupement protecteur » désigne un groupe fonctionnel introduit dans une molécule à partir d'une fonction chimique pour masquer tout ou partie de sa réactivité. Le masquage (protection) d'une fonction chimique sur la molécule améliore ainsi la sélectivité des réactions suivantes. Le terme « protection » ou « protégé » fait ainsi référence à l'état de la molécule après réaction avec un groupement protecteur. Une « molécule biologique protégée » est une molécule biologique qui présente une ou plusieurs fonctions chimiques protégées par des groupements protecteurs. S'agissant d'une enzyme, une enzyme protégée peut alors être inactive. La déprotection fait référence à l'étape de libération de tout ou partie des groupements protecteurs d'une molécule protégée et, de préférence, l'obtention de la molécule dans son état original, avant sa protection par les composés selon l'invention.

Le terme « groupement protecteur thermolabile » désigne un groupement protecteur qui est stable à température entre 15°C et 25°C, et qui est clivé, dissocié ou relargué d'une molécule à laquelle il est lié, par un traitement thermique (étape de chauffage) à une température comprise entre 50 et 100°C, notamment en présence d'un tampon approprié pour le fonctionnement optimal d'une molécule biologique, par exemple, d'une enzyme.

Des groupements protecteurs thermolabiles incluent en particulier ceux décrits par Koukhareva et al, Anal. Chem., 2009, 81, 4955-4962; ou Trinlink Biotechnologies (WO2012/09434 et US8,133,669).

Des exemples de groupements protecteurs thermolabiles incluent de manière générale les amides, les éthers, les esters, les acétals, les carbonates, les thioéthers, les thioesters, thioacétals, thiocarbonates et notamment ceux décrits dans Koukhareva et al, Anal. Chem. 2009, 81, 4955-4962 , ou encore, le monométhoxy trityle (MMT), le diméthoxy trityle (DMT), et/ou le phenoxyacétate, substitué ou non.

Le terme « groupement protecteur acidolabile » désigne un groupement protecteur qui est stable en condition neutre ou basique et qui est clivé, dissocié ou relargué, en condition acide par traitement à un pH inférieur à 6,0, ou encore inférieur à 5,0. Des exemples de groupements protecteurs acidolabiles incluent en particulier le citraconyl, *le tert*-butoxycarbonyl (BOC), le benzyloxycarbonyl, le trityl (Trt), le méthoxytrityl, le diméthoxytrityl, le benzyloxymethyl (Bom) ou encore le t-Butoxymethyl (Bum). D'autres exemples de groupements protecteurs acidolabiles sont décrits notamment dans Chem. Rev., 2009, 109 (6), pp 2455-2504 et plus particulièrement dans les pages 2467 et 2493 selon qu'il s'agit d'un groupement protecteur d'une fonction amine ou hydroxyle.

Le terme « molécule biologique » s'entend au sens large comme incluant l'ensemble des macromolécules synthétisables par des organismes biologiques. En particulier, le terme inclut les polymères d'acides nucléiques et notamment l'ADN ou l'ARN, les polysaccharides, les peptides, polypeptides et les protéines, notamment les enzymes.

Les termes "polypeptide", "peptide", et "protéine" sont interchangeables, et font référence aux polymères ou oligomères d'acides aminés. Les acides aminés d'un tel polymère sont reliés entre eux par une liaison peptidique entre un groupe carboxyl et un groupe amine de deux acides aminés. Une protéine peut comprendre plusieurs polypeptides reliés entre eux par des liaisons non-covalentes et/ou des ponts disulfures.

Au sens de l'invention, le terme "acides aminés" incluent les acides aminés naturels ou non naturels ou leurs dérivés substitués.

### Les Réactifs Pour la Protection de Molécules Biologiques

Les composés selon l'invention ou réactifs pour la protection de molécules biologiques sont des dérivés de l'anhydride isatoïque utiles pour protéger transitoirement (protection « réversible ») les groupements nucléophiles, par exemple les amines, d'une molécule biologique, par exemple une protéine.

Les composés selon l'invention réagissent avec le groupe nucléophile d'une molécule biologique et notamment d'une protéine. Par exemple, ils peuvent réagir avec l'amine ε d'une lysine d'une protéine (ou enzyme) ou l'amine N-terminale d'une protéine (ou enzyme), l'alcool d'une sérine d'une protéine (ou enzyme) ou encore le thiol d'une cystéine d'une protéine pour former respectivement une liaison amide, ester, ou thioester. Parmi les fonctions nucléophiles d'une protéine ou d'une enzyme, de préférence au moins une contribue de manière essentielle au maintien de la conformation de la protéine et/ou de la fonction enzymatique. La protection de ladite fonction nucléophile essentielle (par exemple, une lysine, sérine ou cystéine) entraine ainsi l'inactivation de la protéine ou de l'enzyme.

Avantageusement, la liaison amide, ester et/ou thioester ainsi obtenue entre les molécules biologiques et les composés selon l'invention est particulièrement stable, à basse température ou température ambiante. Des molécules biologiques peuvent être rendues inactives avec les composés selon l'invention par exemple dans des conditions de stockage ou de transport à température ambiante. Plus particulièrement, il est possible de maitriser le point de départ d'une réaction enzymatique en induisant les conditions pour la déprotection d'une enzyme, selon le principe ci-dessous :
- Dans un premier temps, on fait réagir les composés selon l'invention par réaction d'acylation des groupements nucléophiles d'une enzyme avec le composé et on obtient une enzyme protégée.
- Dans un deuxième temps, on déprotège par traitement acide et/ou thermique une fonction nucléophile du composé selon l'invention par clivage d'un groupement protecteur thermolabile et/ou acidolabile présent sur le composé selon l'invention et protégeant ladite fonction nucléophile du composé selon l'invention.
- Dans un troisième temps, on obtient la cyclisation entre la fonction nucléophile déprotégée du composé selon l'invention et le carbonyl de l'amide, l'ester ou thioester obtenu par l'acylation de l'enzyme, qui de façon concomitante permet la réversion de l'acylation, la déprotection de l'enzyme et la restauration de l'activité enzymatique.

Sauf précision ci-après dans le texte qui suit, l'expression « composés selon l'invention » fait référence aux composés de formule (I) ci-dessous ainsi qu'à toutes leurs sous-formules spécifiquement décrites (et en particulier les formules (II) à (VI), les sels de ces composés, leurs stéréoisomères (incluant les diastéréoisomères et les énantiomères), les tautomères et les composés marqués par des isotopes (incluant les substitutions au deutérium et par des isotopes radioactifs).

Le composé selon l'invention de formule (I) ci-dessous 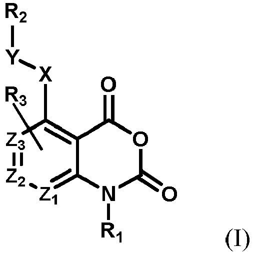 présente en particulier
- un motif anhydride azaisatoïque permettant l'acylation des groupements nucléophiles d'une molécule,
- une fonction nucléophile Y protégée par un groupement thermolabile et/ou acidolabile R₂ et permettant une fois libérée, le clivage de la liaison amide générée par l'acylation,
- un bras de liaison X entre l'anhydride isatoïque et la fonction nucléophile conçu de telle sorte qu'il favorise la cyclisation après déprotection de la fonction nucléophile,
- un groupement protecteur thermolabile et/ou acidolabile R₂ du groupement nucléophile Y permettant de libérer la fonction nucléophile Y lors de l'étape de chauffage et/ou de traitement acide et permettant ainsi la cyclisation intramoléculaire,
- de préférence, un groupement encombrant R₃ permettant un encombrement stérique autour du nucléophile et l'accélération de la cinétique de cyclisation intramoléculaire. En effet il a été découvert de façon fortuite que la présence d'un tel encombrement stérique apportait beaucoup de bénéfice à la cinétique de cyclisation intramoléculaire et donc à la vitesse de déprotection des groupements nucléophiles des molécules biologiques.

Ainsi, la présente invention a pour objet un composé de formule (I) suivante : 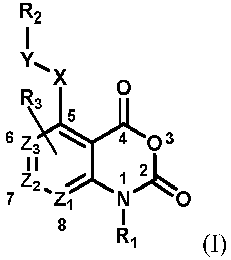 dans laquelle
X est une liaison covalente ou un alkyle en C₁ à C₄,
Y est un radical nucléophile choisi parmi O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-NH-O, O-C(O)-O-NR₄, NH-C(O)-O-NR₄, C(O)-S, et R₄ est H ou un groupe alkyle en C₁ à C₄,
et lorsque Y est choisi parmi O, S et NR4, X n'est pas une liaison,
Z₁, Z₂, Z₃ représentent chacun indépendamment l'un de l'autre, N ou C, de préférence Z₃ représente C, plus préférentiellement Z₃ est C et R₃ est en position Z₃,
R₁ est H, un groupe alkyle en C₁ à C₆, substitué ou non, un alcényle substitué ou non, un groupe aryle substitué ou non, ou un hétérocycle substitué ou non, de préférence R₁ est un groupe méthyle ou éthyle.
R₂ est un groupement protecteur thermolabile et/ou acidolabile,
R₃ est H, un groupe alkyle en C₁ à C₁₂ substitué ou non, par exemple un groupe *iso-*propyle, isobutyle, sec-butyle, *tert*-butyle, isopentyle ou 2,2-diméthylpropyle, un groupe aryle substitué ou non, un hétérocycle substitué ou non, un groupe acyle, un groupe alcényle substitué ou non, un halogène (e.g. F, Cl, Br et I), ou un groupe cyano.

De préférence, un seul des radicaux Z₁, Z₂ et Z₃ est N.

Dans un mode de réalisation particulier, X est un alkyle non substitué en C₁ à C₃, par exemple un méthyle.

Dans un mode de réalisation, R₁ est H, un groupe alkyle en C₁ à C₆, un alcényle, un groupe aryle ou un hétérocycle, lesdits groupes alkyle, alcényle, aryle, ou hétérocycle, étant éventuellement substitués par un ou plusieurs groupes fonctionnels choisis parmi nitrile, cyano, amide, imide, alcoxyle, carbonyle, carboxyle, ester, thioéther, thioester et halogénure.

Dans un mode de réalisation, R₁ est un groupe alkyle en C₁ à C₆, éventuellement substitués par un ou plusieurs groupes fonctionnels choisis parmi nitrile, cyano, amide, imide, alcoxyle, carbonyle, carboxyle, ester, thioéther, thioester et halogénure.

Dans un mode de réalisation, R₂ est un groupement protecteur choisi parmi les amides, les éthers, les esters, les acétals, les carbonates, les thioéthers, les thioesters, les thioacétals, les thiocarbonates, le monométhoxy trityle (MMT), le diméthoxy trityle (DMT), le phenoxyacétate substitué ou non, le citraconyl, *le tert*-butoxycarbonyl (BOC), le benzyloxycarbonyl, le trityl (Trt), le méthoxytrityl, le diméthoxytrityl, le benzyloxymethyl (Bom) ou le t-Butoxymethyl (Bum).

Dans un mode de réalisation, R₃ est H, un groupe alkyle en C₁ à C₁₂, un groupe aryle, un hétérocycle, un groupe acyle, un groupe alcényle, un halogène (e.g. F, Cl, Br et I), ou un groupe cyano, lesdits groupes alkyle, aryle, hétérocycle ou alcényle étant éventuellement substitué par un ou plusieurs groupes fonctionnels choisis parmi les goupes nitrile, cyano, amide, imide, alcoxyle, carbonyle, carboxyle, ester, thioéther, thioester et halogénure. Dans un mode de réalisation, R₃ est un groupe alkyle en C₁ à C₁₂ ou un halogène (e.g. F, Cl, Br et I), ledit groupe alkyle étant éventuellement substitué par un ou plusieurs groupes fonctionnels choisis parmi les goupes nitrile, cyano, amide, imide, alcoxyle, carbonyle, carboxyle, ester, thioéther, thioester et halogénure.

R₃ est placé de sorte à favoriser la cinétique de cyclisation. Par exemple, dans un mode de réalisation, R₃ est de préférence en position 6.

Dans un mode de réalisation particulier, R₃ est un groupe *tert*-butyle, un groupe *iso-*propyle, un groupe cyano ou un atome d'iode.

Dans un mode de réalisation, le nombre d'atome X + Y linéaire, c'est-à-dire le nombre d'atome sur la chaine principale reliant le cycle à R₂ (les différents substituants H ou R₄ n'étant pas pris en compte), est supérieur ou égal à 2, par exemple compris entre 2 et 4. De préférence, Y est choisi parmi O, S et NH. Dans ce cas, X n'est pas une liaison.

Dans un mode de réalisation plus particulièrement préféré, le composé selon l'invention est de formule (II) suivante :

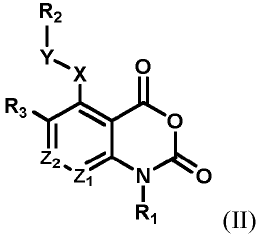

Dans un mode de réalisation spécifique, le groupement thermolabile ou acidolabile R₂ dans les formules (I) et (II) ci-dessus est choisi parmi les groupements *tert*-butoxycarbonyle (BOC), phénoxyacétyle substitué ou non, trityle, méthoxytrityle, diméthoxytrityle ou citraconyle.

Le caractère thermolabile des groupements phénoxyacétate a été démontré par Lebedev A. *et al.* (Koukhareva I., Lebedev A. Anal. Chem., 2009, 81, 4955-4962). Dans un mode de réalisation particulier, le groupement protecteur R₂ est le groupement protecteur thermolabile phénoxyacétyle, substitué ou non, de préférence, il s'agit d'un phénoxyacétyle substitué par un halogène, par exemple le fluorophénoxyacétyle.

L'homme du métier sera à même d'identifier d'autres groupements protecteurs R₂ thermolabiles et/ou acidolabiles particulièrement adaptés à la fonction recherchée comme décrit par exemple dans WO2012/094343, US8,133,669, ou dans les livres suivants :
- Greene's Protective Groups in Organic Synthesis de Peter G. M. Wuts Editeur : Wiley-Blackwell; Édition : 5th Edition (23 décembre 2014) ou
- Protecting Groups de P.J. Kocienski Editeur : Thieme Publishing Group; Édition : 3rd Revised edition (1 janvier 2005).

Dans un autre mode de réalisation particulier du composé selon la formule (II), qui peut être combiné avec les précédents modes de réalisation, R₁ est un groupe méthyle.

Dans un autre mode de réalisation particulier du composé selon la formule (II), éventuellement combiné avec les précédents modes de réalisation, R₃ est un halogène, par exemple, l'iode.

Dans un autre mode de réalisation particulier du composé selon la formule (II), qui peut être combiné avec les précédents modes de réalisation, Z₁ est N et Z₂ est C.

En particulier, l'invention porte sur l'un des composés de structures suivantes, également décrits en exemples :

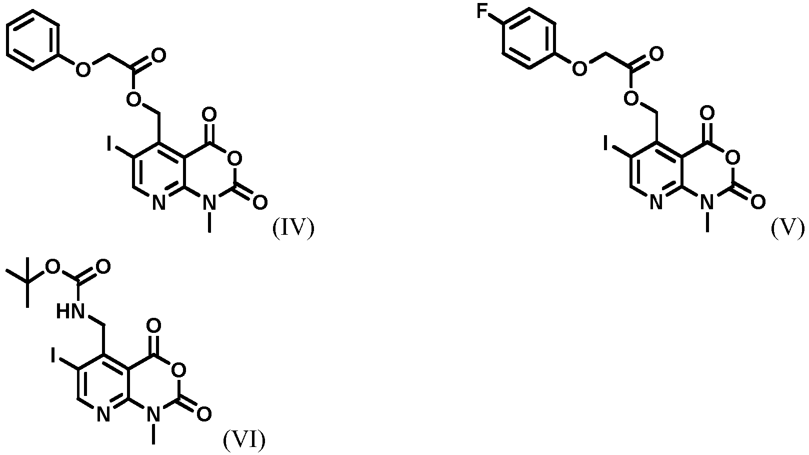

### Procédé de préparation des composés selon l'invention

Les composés selon l'invention peuvent être aisément synthétisés selon les procédés décrits en exemples ou d'autres procédés de synthèse connus dans l'état de la technique.

Par exemple, certains des composés selon l'invention de formule (II) peuvent être synthétisés à partir du composé hydroxylé précurseur **6** dont la synthèse est décrite en exemple 1. Le composé précurseur **6** peut ensuite être modifié par réaction d'un groupement thermolabile ou acidolabile R₂ sur la fonction hydroxyle du précurseur. Il est également possible de faire réagir sur ces composés précurseurs ou leurs dérivés les groupements R₁ et/ou R₃ par des méthodes conventionnelles de chimie. Le composé selon l'invention de formule (II) peut être finalement obtenu par cyclisation par substitution nucléophile, comme décrit par exemple à l'étape (i) du schéma 3 en Exemple 1 ci-dessous pour donner les composés selon l'invention.

### Procédé de protection de molécules biologiques

Les composés selon l'invention sont utiles pour la protection, la modification ou l'inactivation réversible des enzymes, des protéines et plus généralement de molécules biologiques comprenant un ou plusieurs groupes nucléophiles, par exemple, des molécules biologiques comprenant une ou plusieurs amines.

L'invention vise notamment un procédé de préparation d'une molécule biologique comprenant un ou plusieurs groupes nucléophiles protégés, comprenant la mise en contact d'un composé selon l'invention tel que décrit ci-dessus avec une molécule biologique dans des conditions permettant l'acylation d'un ou plusieurs groupes nucléophiles de ladite molécule biologique, pour former une molécule biologique comprenant un ou plusieurs groupes nucléophiles protégés (également appelée ci-après « molécule biologique protégée »).

L'acylation se fait par substitution nucléophile sur une des fonctions carbonyle de l'anhydride avec addition du groupe nucléophile de la molécule biologique et élimination de CO₂.

Dans un mode de réalisation plus particulièrement préféré, la molécule biologique comprend des fonctions amines. En particulier, la molécule biologique est une protéine qui comprend les fonctions ε-amines de résidus lysines ou amine de l'acide aminé terminal. Dans ces modes de réalisation, l'acylation des fonctions ε-amines de résidus lysines ou de l'amine terminale permet de former des liaisons amides très stables, en particulier dans des conditions de stockage à long terme (par exemple sur plus de 24 heures, voire plusieurs jours à température ambiante).

A titre d'illustration, la réaction d'acylation est représentée dans le schéma 1 ci-dessous avec un réactif préféré de formule (II) dans laquelle R₁ est un groupe méthyle, Z₁ est N, Z₂ est C et R₃ est un atome d'iode.

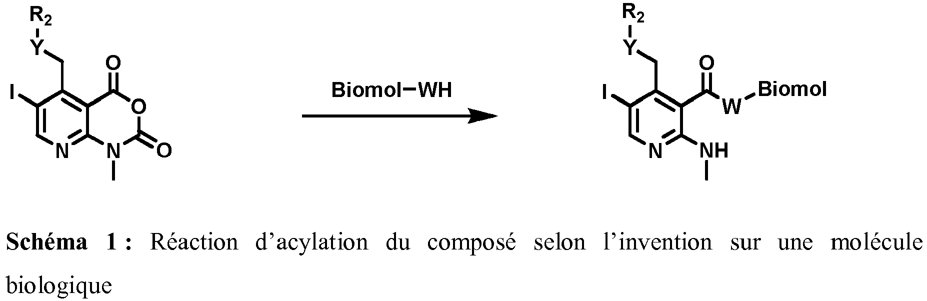

Dans le cas où la molécule biologique comprend plusieurs groupes nucléophiles à protéger, on pourra ajuster la quantité de réactifs utilisée en fonction du degré de modification (protection) souhaité. En particulier, dans le cas d'une enzyme, avantageusement, le degré de modification souhaité correspond au seuil de modification au-delà duquel l'enzyme est inactivée. Ce seuil peut se déterminer de manière empirique par de simples essais.

Le tampon utilisé pour la réaction de protection est en général un tampon avec un pH compris entre 6 et 9, de préférence entre 7 et 9, plus préférentiellement entre 7 et 8. Des exemples de tampons incluent notamment les tampons phosphates, et les tampons non nucléophiles opérant dans cette zone de pH, pouvant contenir jusqu'à 50% de DMSO. De tels tampons sont connus de l'homme du métier comme indiqué dans http://www.sigmaaldrich.com/life-science/core-bioreagents/biological-buffers.html

La réaction d'acylation, notamment d'acylation d'une enzyme avec au moins un composé de formule (I) ou (II) est de préférence obtenue dans un tampon phosphate, par exemple à pH 7,4.

Le procédé de protection est de préférence mis en œuvre à une température comprise entre 4°C et 40°C, par exemple une température comprise entre 4°C et 25°C.

Les molécules biologiques comprenant un ou plusieurs groupes nucléophiles protégés, susceptibles d'être obtenues par le procédé ci-dessus font également partie de la présente invention. En particulier, une enzyme peut comprendre plusieurs groupements amines protégées du fait de la présence de multiples lysines dans sa séquence polypeptidique, ou encore des groupes OH ou SH du fait de la présence de sérine ou cystéine respectivement.

L'invention porte par conséquent sur une molécule biologique protégée, et représentée par la formule (III) suivante : 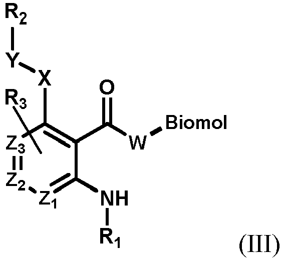 dans laquelle
Biomol est une molécule biologique,
W est un groupe nucléophile de la molécule biologique, de préférence NH, S ou O, X est une liaison covalente ou un alkyle en C₁ à C₄,
Y est un radical nucléophile choisi parmi O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-NH-O, O-C(O)-O-NR₄, NH-C(O)-O-NR₄, C(O)-S, de préférence O, S, NR₄, O-NR₄, NH-O, ou NH-NR₄, et R₄ est H ou un groupe alkyle en C₁ à C₄
et lorsque Y est choisi parmi O, S et NR₄, X n'est pas une liaison,
Z₁, Z₂, Z₃ représentent chacun indépendamment l'un de l'autre, N ou C, de préférence Z₃ représente C, plus préférentiellement Z₃ est C et R₃ est en position Z₃,
R₁ est H, un groupe alkyle en C₁ à C₆, substitué ou non, un alcényle substitué ou non, un groupe aryle substitué ou non, ou un hétérocycle substitué ou non, de préférence R₁ est un groupe méthyle ou éthyle,
R₂ est un groupement protecteur thermolabile et/ou acidolabile,
R₃ est H, un groupe alkyle en C₁ à C₁₂ substitué ou non, par exemple un groupe iso-propyl, isobutyl, sec-butyl, tert-butyl, isopentyl ou 2,2-diméthylpropyl, un groupe aryle substitué ou non, un hétérocycle substitué ou non, un groupe acyle, un groupe alcényle substitué ou non, un halogène (e.g. F, Cl, Br et I), ou un groupe cyano.

Bien entendu, lorsque la molécule biologique comprend plusieurs groupes nucléophiles, par exemple une protéine comprenant plusieurs lysines, une même molécule biologique peut comprendre tout ou partie de ces groupes nucléophiles ainsi protégés par acylation avec le réactif selon l'invention.

En particulier, lorsque le réactif de formule (II) est mis à réagir avec une molécule biologique comprenant un ou plusieurs groupes nucléophile, on obtient une molécule biologique protégée de formule (VII) suivante :

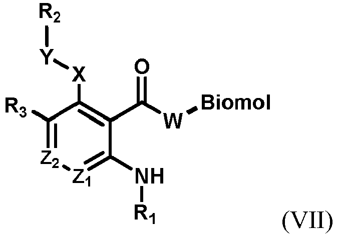

En choisissant l'un des réactifs de formule (IV), (V) et (VI) ci-dessus, on obtient des molécules biologiques protégées selon l'une des formules (VIII), (IX) et (X) respectivement.

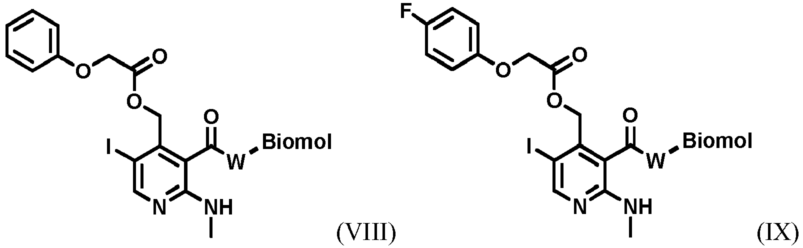

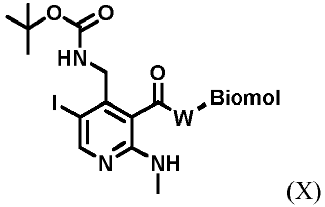

Dans un mode de réalisation spécifique, la molécule biologique (ou Biomol) est choisie parmi les enzymes.

Le procédé est applicable à tout type d'enzyme. Parmi les enzymes d'intérêt, on citera plus particulièrement les enzymes utilisées dans les techniques de biologie moléculaire, pour l'ingénierie génétique ou la polymérisation des acides nucléiques, et plus particulièrement les enzymes utilisées dans les techniques de diagnostic in vitro.

Ces enzymes comprennent, à titre d'illustration, les lipases, les protéases, les glycolases ou les nucléases.

En particulier, les enzymes d'intérêt comprennent les enzymes de restriction, les ligases, les ARN polymérases, les ADN polymérases, tels que les l'ADN polymérase I, II ou III, ou l'ADN polymérase α, β, ou γ, la terminal désoxynucléotidyl transferase (TdT) ou la télomérase, l'ARN polymérase ADN dépendante, la primase, ou encore l'ADN polymérase ARN dépendante (transcriptase inverse).

Dans un mode de réalisation plus particulièrement préféré, la molécule biologique selon l'invention est une enzyme destinée à être utilisée dans une réaction de polymérisation d'acide nucléique, par exemple une ADN polymérase. Les polymérases utilisables pour la polymérisation des acides nucléiques sont bien connues de l'homme du métier. La réaction de polymérisation est en particulier une réaction de polymérisation dans le cadre d'une amplification par polymérisation en chaine (PCR).

Dans un mode de réalisation, une enzyme d'intérêt mise en œuvre dans le procédé de protection selon l'invention est choisie parmi les polymérases suivantes : l'ADN polymérase T7, l'ADN polymérase Kornberg, l'ADN polymérase Klenow, l'ADN polymérase Taq, l'ADN polymérase Micrococcal, l'ADN polymérase alpha, l'ADN polymérase Pfu, la transcriptase inverse AMV, la transcriptase inverse MMLV, l'ARN polymérase d'E.coli, l'ARN polymérase SP6, T3 ou T7. En particulier, dans un mode de réalisation, des ADN polymérases thermostables et/ou présentant une activité exonucléase (par exemple 3'5' exonucléase) sont utilisées dans le procédé selon l'invention pour préparer des polymérases protégées.

Ces polymérases ainsi protégées sont plus particulièrement utiles pour des applications de démarrage à chaud « Hot-Start ». La protection des polymérases empêche l'amplification non-spécifique d'ADN à basse température et favorise ainsi une meilleure efficacité de la réaction d'amplification, l'enzyme étant déprotégée uniquement à haute température, par clivage du groupement thermolabile.

Parmi ces polymérases thermostables, on citera notamment celles qui ne sont pas sujettes à une dénaturation de leur structure et/ou une inactivation de leur activité enzymatique entre 50 °C et 100°C, celles qui fonctionnent et sont actives une fois qu'elles sont déprotégées, entre 50°C et 100°C. Plus particulièrement, les polymérases : TAQ Polymerase et KLen TAQ polymerase.

D'autres enzymes thermostables peuvent être dérivées des organismes biologiques suivants : Thermus antranikianii, Thermus aquaticus, Thermus caldophilus, Thermus chliarophilus, Thermus filiformis, Thermus flavus, Thermus igniterrae, Thermus lacteus, Thermus oshimai, Thermus ruber, Thermus rubens, Thermus scotoductus, Thermus silvanus, Thermus species Z05, Thermus species sps 17, Thermus thermophilus, Thermotoga maritima, Thermotoga neapolitana, Thermosipho africanus, Anaerocellum thermophilum, Bacillus caldotenax, Bacillus stearothermophilus, etc..

### Procédés d'utilisation des composés selon l'invention

Les molécules biologiques protégées selon la présente invention peuvent être avantageusement déprotégées par un simple traitement thermique et/ou acide selon groupement R₂ choisi.

Le principe de la déprotection par traitement thermique ou acide est décrit dans le schéma 2 qui suit avec une molécule biologique (enzyme) protégée, selon la formule (VII) dans laquelle R₁ est un groupe méthyle, Z₁ est N, Z₂ est C et R₃ est un atome d'iode:

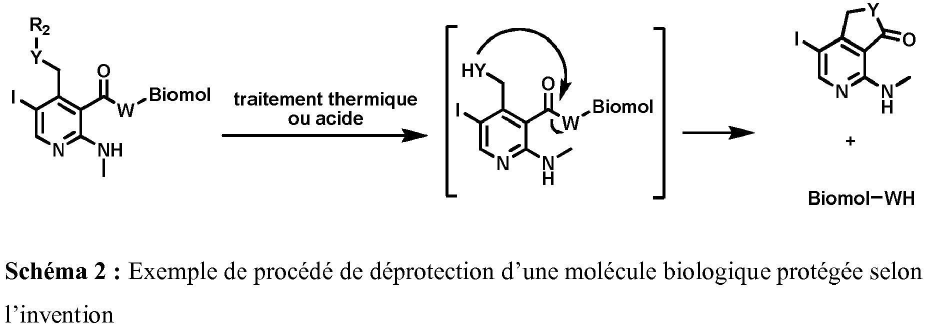

Un traitement thermique ou acide permet de libérer la fonction nucléophile du composé selon l'invention par clivage du groupement thermolabile ou acidolabile. Cette fonction nucléophile ainsi libérée peut alors permettre la réversion de l'acylation par cyclisation et déprotection de la molécule biologique. Avantageusement, le sous-produit généré par la réaction de déprotection n'est pas réactif. De plus, la présente réaction de déprotection ne nécessite pas l'emploi d'un tampon Tris, ou d'un tampon qui génère des conditions acides à haute température. En particulier, l'étape de déprotection peut être effectuée par traitement thermique dans des conditions de pH compris entre 6,5 et 9,5, de préférence entre 8 et 9,0, plus préférentiellement encore entre 8,5 et 9,0.

Ainsi, sont également décrits des procédés de déprotection des groupes nucléophiles d'une molécule biologique protégée par les composés selon l'invention, ledit procédé comprenant une étape de clivage du ou des groupements thermolabiles et/ou acidolabiles R₂, par traitement thermique et/ou acide respectivement, et la déprotection concomitante des groupes nucléophiles.

Un des avantages du procédé décrit ci-dessus est qu'il permet d'inactiver des enzymes. Par « inactivation » il faut comprendre que l'activité catalytique d'une enzyme protégée par le procédé de l'invention est fortement réduite voire nulle par rapport à son activité catalytique avant protection dans des conditions optimales d'activité.

Le procédé de déprotection décrit ci-dessus permet alors de restaurer l'activité enzymatique des enzymes protégées.

Ainsi, l'invention concerne également les utilisations d'un composé selon l'invention pour l'inactivation réversible d'une enzyme. Dans un mode préféré, le composé selon l'invention est utilisé pour l'inactivation réversible d'une enzyme destinée à être utilisée dans une réaction d'amplification d'acide nucléique, par exemple une polymérase.

L'invention est particulièrement appropriée pour des applications de démarrage à chaud (« Hot Start ») des enzymes, par exemple les polymérases utilisées dans une réaction d'amplification d'acide nucléique (réaction dites « PCR »).

Par conséquent, l'invention vise également un procédé d'amplification d'un acide nucléique comprenant (i) la mise en œuvre d'une enzyme polymérase protégée ou inactivée par les composés selon l'invention, (ii) au moins une étape pour la déprotection de la polymérase, par exemple par un traitement thermique à une température permettant le clivage du ou des groupements thermolabiles R₂, (iii) une étape d'amplification d'acide nucléique à l'aide de la polymérase déprotégée à l'étape (ii).

Dans un mode de réalisation spécifique, le procédé d'amplification est mis en œuvre à l'aide d'une ADN polymérase, telle que la Taq polymérase, la Pfu polymérase, la T7 polymérase, le fragment Klenow d'ADN polymérase d'E. coli et/ou une transcriptase inverse, ou tout autre polymérase telle que décrite plus haut.

Dans un autre mode de réalisation qui peut être combiné avec le précédent, le procédé d'amplification est une réaction d'amplification en chaine par polymérase (réaction PCR), bien connue de l'homme du métier. Le protocole PCR comprend par exemple, 20 à 40 cycles, chaque cycle comprenant au moins (i) une phase de dénaturation de l'ADN à amplifier à une température généralement comprise entre 90°C et 95°C, (ii) une phase d'hybridation des amorces avec l'ADN à amplifier à une température généralement comprise entre 55°C et 65°C et (iii) une phase d'extension à une température généralement comprise entre 68°C et 75°C.

La polymérase protégée selon l'invention est dans ce cas de préférence une polymérase thermostable protégée, par exemple une *Taq* (Thermus aquaticus) polymérase protégée, une Pfu (Pyrococcus furiosus) polymérase protégée, Vent ou Tli (Thermococcus litoralis) polymérase protégée ou leurs variantes, notamment recombinantes. De préférence, un nombre suffisant d'amines de ladite polymérase sont protégées de sorte que la polymérase protégée est inactive ou quasi-inactive à température ambiante et peut être déprotégée à une température équivalente ou supérieure à la température d'hybridation des amorces (primer) utilisées pour l'amplification PCR.

Les enzymes et polymérases protégées selon l'invention sont également avantageusement mises en œuvre dans des variantes de procédés d'amplification nucléique par PCR. On citera en particulier la PCR emboitée (Nested PCR), les PCR quantitatives (ou qPCR), semi-quantitatives ou temps réel, les PCR « error-prone », ou la PCR après transcription inverse (RT-PCR).

### Kits et Trousses de mise en œuvre

L'invention vise également des kits ou trousses de mise en œuvre comprenant les enzymes protégées selon l'invention, et éventuellement des réactifs, des tampons, des contrôles et/ou une notice d'instructions de mise en œuvre.

En particulier, l'invention concerne une trousse pour l'amplification d'acide nucléique en démarrage à chaud « Hot-start », comprenant
i) une ADN polymérase thermostable protégée selon l'invention, par exemple de formule (III), (VII), (VIII), (IX) ou (X) dans lesquelles Biomol est une ADN polymérase thermostable,
ii) le cas échéant, des réactifs de détection d'acide nucléique, par exemple un réactif fluorescent,
iii) le cas échéant, un tampon, des dNTP...

Ces kits sont utiles notamment pour la mise en œuvre de procédés d'amplification tel que décrits ci-dessus.

L'invention sera mieux comprise à l'aide des exemples détaillés ci-dessous et au regard des figures annexées.

### DESCRIPTION DES FIGURES

**Figure 1** : Suivi par HPLC du clivage du dérivé aza-anthranylate phénoxyacétate **10** et de la libération de phényléthylamine.
**Figure 2** : Suivi par HPLC du clivage du dérivé aza-anthranylate fluoro-phénoxyacétate **14** et de la libération de phényléthylamine.
**Figure 3** **:** Suivi par HPLC du clivage du dérivé aza-anthranylate N -Boc **19** et de la libération de phényléthylamine.
**Figure 4****:** Acylation de l'hémoglobine par le composé **13** utilisé à différentes concentrations.
**Figure 5****:** Réversion de l'acylation de l'hémoglobine suite à un traitement thermique.
Figure du haut (A) : Hémoglobine dans GuHC 8M
Figure du milieu (B) : Hémoglobine acylée par le composé **13** puis reprise dans GuHCL 8M
Figure du bas (C) : Hémoglobine acylée par le composé **13** puis chauffée à 95°C dans du Tris pH9 et reprise dans GuHCl 8M.
**Figure 6****:** Inactivation de la *Taq* polymerase suite à son acylation par le composé **13** et restauration de son activité après traitement thermique à 95°C pendant 15 min dans des conditions de PCR (expériences réalisées en duplicat, la moyenne est représentée). En pointillé clair à gauche : sans activation ; en pointillé grisé à droite : après 15 minutes d'activation à 95°C.

### EXEMPLES

Dans les exemples décrits ci-après, les abréviations suivantes seront utilisées :
- ACN : Acétonitrile,
- AcOEt : acétate d'éthyle,
- Boc₂O : dicarbonate de di-*tert*-butyle,
- CCM : chromatographie sur couche mince,
- CDCl₃ : Chloroforme deutéré,
- d : doublet,
- DCM : dichlorométhane,
- dd : doublet dédoublé,
- DMF : diméthylformamide,
- DMSO: diméthylsulfoxide,
- DMSO-*d₆* : diméthylsulfoxide deutéré,
- Eau MilliQ : Eau ultrapure (Millipore, Molsheim, France),
- EDC : N-Ethyl-N'-(3-diméthylaminopropyl)carbodiimide
- éq : équivalents,
- EP : éther de pétrole,
- Et₂O : éther diéthylique,
- HPLC : chromatographie liquide haute performance,
- LCMS : Appareil de chromatographie liquide couplé à un spectromètre de masse
- HOBt : hydroxybenzotriazole,
- IA : anhydride isatoïque,
- m : multiplet,
- nd : non déterminé,
- NIS : *N*-iodosuccinimide,
- q : quadruplet,
- Rdt : rendement,
- Rf ou TR : temps de rétention,
- RMN : résonance magnétique nucléaire,
- s : singulet,
- t : triplet,
- ta : température ambiante
- TBDMS : *tert*-butyldiméthylsilyle
- TEA : triéthylamine
- THF : tétrahydrofurane.

### Conditions générales

Les conditions générales pour l'analyse et la synthèse des composés chimiques utilisées dans les exemples ci-après sont décrites ci-dessous:
Les analyses LC-MS ont été effectuées avec une chaîne HPLC WATERS Alliance 2795 équipée d'un détecteur à barrette de diodes PDA 996 (Waters), d'un détecteur par spectrométrie de masse ZQ 2000 (Waters), d'un logiciel Empower version 2 et d'une colonne WATERS XTerra MS C18 (4,6 x 30 2,5 µm) utilisée avec un débit de 1 ml/minute à 30°C (détection à 260 nm ou en max plot). Le spectromètre de masse ZQ 2000 possède une source d'ionisation Electrospray. Les ionisations ont été réalisées en mode positif avec une tension de cône de 20V et une tension au niveau du capillaire de 3,5kV.

Les conditions utilisées pour les analyses HPLC sont les suivantes:

| **Eluant A** | **Eluant B** | **Eluant C (Formiate d'ammonium : AF)** | **Gradient linéaire** |
|---|---|---|---|
| Eau milliQ | ACN | AF 500 mM, pH 7 | 98% de A / 0% de B à 24% de A /74% de B en 18 min avec 2% de l'éluant C en mode isocratique |

Les spectres RMN ont été enregistrés sur un spectromètre Jeol Lambda 400 MHz. Les déplacements chimiques (δ) sont donnés en ppm par rapport au pic du solvant pris comme référence interne (CDCl₃ : 7,26 ppm ; DMSO-d₆ : 2,49 ppm). Les spectres sont décrits avec les abréviations ci-dessus : s, d, t, q, qu et m. Les constantes de couplage (J) sont exprimées en hertz (Hz).

Les chromatographies sur colonne ont été réalisées sur gel de silice Macherey-Nagel Kieselgel 60, 0,063-0,2 mm / 70-230 mesh ou Merck LiChroprep® RP-18 40-63 µm.

Les analyses par chromatographie sur couche mince ont été effectuées sur des plaques Macherey-Nagel POLYGRAM® SIL G/UV254, 0,20 mm ou ALUGRAM® RP-18 W/UV254 0,15 mm.

### Exemple 1 : Synthèse d'une molécule d'anhydride aza-isatoique selon l'invention dotée d'un groupement protecteur thermolabile O-phénoxyacétate (9) (correspondant au composé IV)

On décrit dans cet exemple la synthèse d'un composé selon l'invention (selon le schéma 3). Un premier composé hydroxylé précurseur **6** est tout d'abord synthétisé en 6 étapes. Il peut servir de composé de départ pour la synthèse d'autres composés selon l'invention. Le composé **6** est alors phénoxyacétylé pour donner le composé **7,** puis iodé pour donner le composé **8** et enfin cyclisé afin d'obtenir le composé **9** aza-isatoique attendu à même de réagir avec une protéine ou tout autre molécule biologique pour la protéger de façon transitoire.

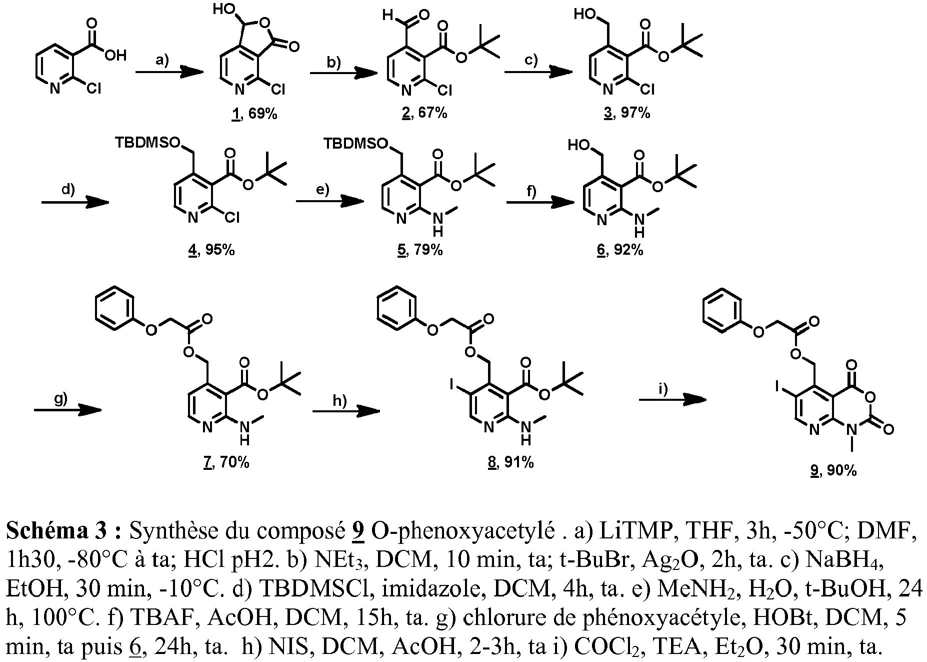

### Exemple 1.1: Synthèse du 4-chloro-1-hydroxyfuro[3,4-c]pyridine-3(1H)-one (1)

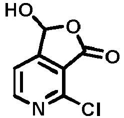

**C₇H₄ClNO₃**

**M = 185,56 g/mole**

Dans un tube de Shlenk de 500 mL sous azote, 19,30 mL de 2,2,6,6-tétramethylpiperidine (114,24 mmoles, 3 éq) sont mis en solution dans 150 mL de THF. A -10°C, 60,13 mL de *n*-BuLi (1.9 M dans l'hexane, 114,24 mmoles, 3 éq) sont ajoutés et le mélange réactionnel est laissé sous agitation pendant 10 min. A -80 °C, 6,00 g d'acide 2-chloronicotinique (38,08 mmoles, 1 éq) sont ajoutés et la réaction est laissée sous agitation pendant 3h à - 50°C. 17,69 mL de DMF (228,48 mmoles, 6 éq) sont alors introduits à -80°C et le mélange réactionnel est laissé sous agitation pendant 1h30. Après retour à température ambiante, 100 mL d'eau sont ajoutés et la solution est extraite avec de l'AcOEt (3x150 mL). La phase aqueuse est ensuite acidifiée à pH 2 avec une solution d'HCl concentrée puis extraite avec de l'AcOEt (3x200 mL). Les phases organiques sont alors rassemblées, séchées sur MgSO₄, filtrées et enfin évaporées. L'huile obtenue est ensuite purifiée sur une colonne de gel de silice en utilisant un gradient d'éluant (DCM à DCM/AcOEt 85/15).

Le produit final est obtenu sous forme de poudre blanche avec un rendement de 69% (4,88 g, 26,30 mmoles).
**Pf** = 191-193°C; **IR (KBr):** v, 3100 (OH), 2913, 2766, 1776 (C=O), 1609, 1584, 1408, 1194, 1136, 1093, 1047, 925, 755 cm⁻¹; **RMN ¹H (400MHz, DMSO-*d₆*):** δ 6,67 (bs, 1H); 7,79 (d, 1H, *³J* = 5,0 Hz); 8,50 (bs, 1H); 8,74 (d, 1H, *³J* = 5,0 Hz); **RMN ¹³C (100 MHz, DMSO-*d₆*):** δ 96,9; 118,8; 120,4; 147,0; 154,6; 159,3; 164,6.

### Exemple 1.2: Synthèse du 2-chloro-4-formylnicotinate de tert-butyle (2)

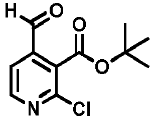

**C₁₁H₁₂ClNO₃**

**M = 241,67 g/mole**

Dans un ballon de 250 mL, à température ambiante, sont introduits 3,50 g de **1** (18.86 mmoles, 1 éq), 35 mL de DCM et 2,62 mL de TEA (18.86 mmoles, 1 éq). Le mélange est laissé sous agitation pendant 10 min à température ambiante. 8,54 mL de *t*-BuBr (75.45 mmoles, 4 éq) et 8,74 g d'Ag₂O (37.72 mmoles, 2 éq) sont alors ajoutés par portions à 0°C. La réaction est laissée sous agitation à température ambiante pendant 2h. Le mélange est filtré sur célite, évaporé et purifiée sur une colonne de gel de silice en utilisant un gradient d'éluant (cyclohexane/AcOEt 95/5 à cyclohexane/AcOEt 90/10). Le produit final est obtenu sous forme de poudre blanche avec un rendement de 67% (3.07 g, 12,70 mmoles).
**Pf** = 97-99°C; **IR (KBr):** v, 3077, 1732 (C=O), 1708 (C=O), 1578, 1370, 1294, 1261, 1178, 1133, 847 cm⁻¹; **RMN ¹H (400MHz, CDCl₃):** δ 1,64 (s, 9H); 7,65 (d, 1H, *³J* = 5,0 Hz); 8,65 (d, 1H, *³J* = 5,0 Hz); 10,06 (s, 1H); **RMN ¹³C (100 MHz, CDCl₃):** δ 27,9 (3C); 85,1; 121,0; 130,1; 140,6; 149,1; 150,9; 163,0; 188,2.

### Exemple 1.3: Synthèse du 2-chloro-4-(hydroxyméthyl)nicotinate de tert-butyle (3)

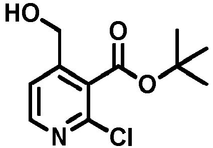

**C₁₁H₁₄ClNO₃**

**M = 243,69 g/mole**

Dans un ballon de 100 mL, 3,00 g de **2** (12,41 mmoles, 1 éq) sont mis en solution dans 35 mL d'EtOH. A -10°C, 0,51 g de NaBH₄ (13,65 mmoles, 1,1 éq) sont ajoutés par portions et le mélange réactionnel est laissé sous agitation pendant 30 min. 50 mL d'eau sont ensuite ajoutés et la solution est extraite avec du DCM (3x75 mL). Les phases organiques sont alors rassemblées, lavées avec une solution saturée en NaCl (2x50 mL), séchées sur MgSO₄ et évaporées.

Le produit final est obtenu sous forme de poudre blanche avec un rendement de 97% (2.94 g, 12,06 mmoles).
**Pf** = 122-124°C; **IR (KBr):** v, 3263 (OH), 3003, 2980, 1717 (C=O), 1592, 1387, 1365, 1299, 1170, 1131, 1080, 1063, 869, 846 cm⁻¹; **RMN ¹H (400MHz, CDCl₃):** δ 1,60 (s, 9H); 2,89 (t, 1H, *³J* = 6,3 Hz); 4,69 (d, 2H, *³J* = 6,3 Hz); 7,39 (d, 1H, *³J* = 5,0 Hz); 8,37 (d, 1H, *³J* = 5,0 Hz); **RMN ¹³C (100 MHz, CDCl₃)**: δ 27,9 (3C); 61,3; 84,3; 120,6; 128,6; 147,2; 149,8; 150,8; 164,8.

### Exemple 1.4: Synthèse du 4-(((tert-butyldiméthylsilyl)oxy)méthyl)-2-chloronicotinate de tert-butyle (4)

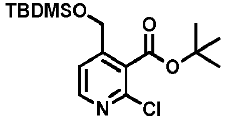

**C₁₇H₂₈ClNO₃Si**

**M = 357,95 g/mole**

Dans un ballon de 100 mL à température ambiante, sont introduits 2,25 g de 3 (9,23 mmoles, 1 éq), 30 mL de DCM, 1,88 g d'imidazole (27,70 mmoles, 3 éq) et 2,78 g de TBDMSC1 (18,47 mmoles, 2 éq). Le mélange réactionnel est laissé sous agitation pendant 4h à température ambiante. 50 mL d'eau sont ensuite ajoutés et la solution est extraite avec du DCM (3x75 mL). Les phases organiques sont alors rassemblées, lavées avec une solution saturée en NaCl (2x50 mL), séchées sur MgSO₄ et évaporées. Le brut réactionnel est ensuite purifié sur une colonne de gel de silice en utilisant un gradient d'éluant (EP à EP/Et₂O 95/5). Le produit final est obtenu sous forme d'huile incolore avec un rendement de 95% (3,15 g, 8,80 mmoles).
**IR (KBr):** v, 2956, 2931, 2859, 1717 (C=O), 1584, 1369, 1291, 1259, 1168, 1127, 840 cm⁻¹; **RMN ¹H (400MHz, CDCl₃):** δ 0,10 (s, 6H); 0,94 (s, 9H); 1,60 (s, 9H); 4,74 (s, 2H); 7,50 (d, 1H, *³J* = 5,0 Hz); 8,39 (d, 1H, *³J* = 5,0 Hz); **RMN ¹³C (100 MHz, CDCl₃):** δ -5,6 (2C); 18,2; 25,7 (3C); 27,9 (3C); 61,1; 83,6; 119,7; 127,6; 146,7; 149,7; 151,0; 164,1.

### Exemple 1.5: Synthèse du 4-(((tert-butyldiméthylsilyl)oxy)méthyl)-2-méthylamino) nicotinate de tert-butyle (5)

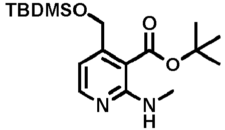

**C₁₈H₃₂N₂O₃Si**

**M = 352,54 g/mole**

Dans un tube scellé à température ambiante sont introduits 4,00 g de **4** (11,17 mmoles, 1 éq), 15 mL de *t*-BuOH et 4,83 mL de MeNH₂ (40% w/w dans H₂O, 55,87 mmoles, 5 éq). Le mélange réactionnel est alors laissé sous agitation pendant 24h à 100°C. Après évaporation, 50 mL d'eau sont ensuite ajoutés et la solution est extraite avec du DCM (3x75 mL). Les phases organiques sont alors rassemblées, séchées sur MgSO₄ et évaporées. Le brut réactionnel est ensuite purifié sur une colonne de gel de silice en utilisant un gradient d'éluant (EP à EP/Et₂O 90/10).

Le produit final est obtenu sous forme d'huile incolore avec un rendement de 79% (3,10 g, 8,79 mmoles).
**IR (KBr):** v, 3377 (N-H), 2931, 2857, 1732 (C=O), 1584, 1370, 1291, 1259, 1190, 1169, 1127, 1112, 839 cm⁻¹; **RMN ¹H (400MHz, CDCl₃):** δ 0,11 (s, 6H); 0,96 (s, 9H); 1,59 (s, 9H); 3,02 (d, 3H, *³J* = 4,9 Hz); 4,90 (s, 2H); 6,97 (d, 1H, *³J* = 5,2 Hz); 7,87 (bs, 1H); 8,25 (d, 1H, *³J* = 5,2 Hz); **RMN ¹³C (100 MHz, CDCl₃)**: δ -5,4 (2C); 18,4; 25,9 (4C); 28,4 (3C); 63,9; 82,2; 104,9; 109,0; 151,8; 154,6; 159,5; 167,8.

### Exemple 1.6: Synthèse du 4-(hydroxyméthyl)-2-(méthylamino)nicotinate de tert-butyle (6)

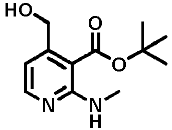

**C₁₂H₁₈N₂O₃**

**M = 238,28 g/mole**

Dans un ballon de 100 mL, 3,00g de **5** (8,51 mmoles, 1 éq) sont mis en solution dans 30 mL de DCM. 0,97 mL d'AcOH (17,02 mmoles, 2 éq) et 17,02 mL de TBAF (1M dans le THF, 17,02 mmoles, 2 éq) sont ajoutés simultanément. La réaction est laissée sous agitation à température ambiante pendant 15h. 70 mL d'eau sont ensuite ajoutés et la solution est extraite avec du DCM (3x70 mL). Les phases organiques sont alors rassemblées, séchées sur MgSO₄ et évaporées. Le brut réactionnel est ensuite purifié sur une colonne de gel de silice en utilisant un gradient d'éluant (EP/AcOEt 80/20 à EP/AcOEt 60/40).

Le produit final est obtenu sous forme de poudre blanche avec un rendement de 92% (1,86 g, 7,81 mmoles).
**IR (KBr):** v, 3348 (N-H), 3242 (O-H), 2980, 2944, 1667 (C=O), 1596, 1557, 1530, 1367, 1242, 1165, 1126, 1074, 1061, 805 cm⁻¹; **RMN ¹H (400MHz, CDCl₃)**: δ 1,60 (s, 9H); 3,01 (d, 3H, *³J* = 4,9 Hz); 4,73 (s, 2H); 6,73 (d, 1H, *³J* = 5,2 Hz); 7,58 (bs, 1H); 8,22 (d, 1H, *³J* = 5,2 Hz); **RMN ¹³C (100 MHz, CDCl₃)**: δ 28,3 (3C); 28,4; 64,0; 83,0; 106,4; 110,7; 151,8; 153,2; 159,2; 167,5.

### Exemple 1.7: 2-(méthylamino)-4-((2-phénoxyacétoxy)méthyl)nicotinate de tert-butyl (7)

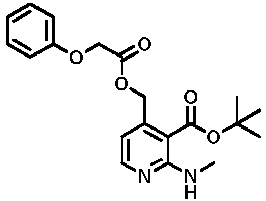

**C₂₀H₂₄N₂O₅**

**M = 372,41 g/mole**

Dans un ballon de 50 mL, 204 µL de chlorure de phénoxyacétyle (1,47 mmoles, 1 éq) sont mis en solution dans 5 mL de DCM. 198 mg d'HOBt (1,47 mmoles, 1éq) sont ajoutés et le milieu réactionnel est laissé sous agitation à température ambiante. Après 10 min, 350 mg de 6 (1,47 mmol, 1 éq) sont ajoutés et la réaction est ensuite agitée à température ambiante pendant 24h. 40 mL d'eau sont ensuite ajoutés et la solution est extraite avec de l'Et₂O (4x30 mL). Les phases organiques sont alors rassemblées, séchées sur MgSO₄ et évaporées. Le brut réactionnel est ensuite purifié sur une colonne de gel de silice en utilisant un gradient d'éluant (EP à EP/AcOEt 8/2).

Le produit final est obtenu sous forme de poudre blanche avec un rendement de 70% (385 mg, 1,03 mmoles).
**Pf =** 149-151°C; **IR** (KBr): v, 3365, 2970, 1760, 1668, 1583, 1192, 752; **RMN ¹H** (CDCl₃, 400 MHz): δ 1,59 (s, 9H); 3,01 (d, 3H, *³J* = 4,9 Hz); 4,75 (s, 2H); 5,45 (s, 2H); 6,53 (d, 1H, *³J* = 5,1 Hz); 6,94 (m, 2H); 7,01 (t, 1H, *³J* = 7,3 Hz); 7,30 (m, 2H); 7,89 (d, 1H, *³J* = 4,2 Hz); 8,18 (d, 1H, *³J* = 5,12 Hz); **RMN ¹³C** (CDCl₃, 100 MHz): δ 28,3 (4C); 65,2; 65,4; 83,1; 105,6; 108,9; 114,6 (2C); 121,8; 129,6 (2C); 147,4; 152,0; 157,6; 159,6;. 167,1; 168,6.

### Exemple 1.8: 5-iodo-2-(méthylamino)-4-((2-phénoxyacétoxy)méthyl)nicotinate de tert-butyle (8)

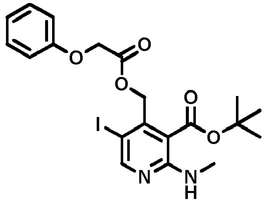

**C₂₀H₂₃IN₂O₅ (M = 498,31 g/mole)**

Dans un ballon de 25 mL, sont introduits 280 mg de **7** (0,75 mmoles, 1 éq), 5 mL de DCM, 253 mg de NIS (1,12 mmoles, 1,5 éq) et 500 µL d'acide acétique. Le mélange réactionnel est laissé sous agitation pendant 3h à température ambiante. Le mélange réactionnel est ensuite neutralisé avec 20 mL d'une solution aqueuse saturée de thiosulfate de sodium, repris dans 20 mL d'une solution saturée de NaHCO₃, puis extrait avec de l'Et₂O (4×30 mL). Les phases organiques sont alors rassemblées, séchées sur MgSO₄ et évaporées. Le brut réactionnel est ensuite purifié sur une colonne de gel de silice en utilisant un gradient d'éluant (EP à EP/AcOEt 9/1).

Le produit final est obtenu sous forme de poudre jaune avec un rendement de 91% (340 mg, 0,68 mmoles).
**Pf =** 127-129°C; **IR** (KBr) v, 3424, 2934, 1755, 1704, 1576, 1193, 752; **RMN ¹H** (CDCl₃, 400 MHz): δ 1,56 (s, 9H); 2,97 (d, 3H, *³J* = 4,6 Hz); 4,64 (s, 2H); 5,4 (s, 2H); 6,90 (d, 2H, *³J* = 7,8 Hz); 6,99 (t, 1H, *³J* = 7,3 Hz); 7,27 (m, 2H); 8,50 (s, 1H); le signal N-H est manquant; **RMN ¹³C** (CDCl₃, 100 MHz): δ 28,1 (3C); 28,4; 65,0; 68,7; 82,5; 83,7; 112,7; 114,6 (2C); 121,8; 129,5 (2C); 144,6; 157,5; 157,7; 158,3; 166,5; 168,3.

### Exemple 1.9: (6-iodo-1-méthyl-2,4-doxo-2,4-dihydro-1H-pyrido[2,3-d][1,3]oxazine-5-yl)méthyl 2-phénoxyacétate (9)

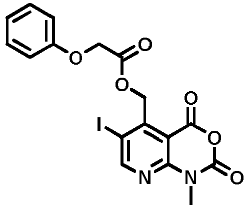

**C₁₇H₁₃IN₂O₆**

**M = 468,20 g/mole**

Dans un ballon de 50 mL sous atmosphère d'azote, 300 mg de **8** (0,60 mmoles, 1 éq) sont mis en solution dans 10 mL de DCM. 951 µL de phosgène (20% dans le toluène, 1,80 mmoles, 3 éq) et 251 µL de TEA (1,80 mmoles, 3 éq) sont ajoutés simultanément et la réaction est laissée sous agitation à température ambiante. Cette opération est répétée à trois reprises pour convertir la totalité de la matière première. 40 mL d'eau sont ensuite ajoutés et la solution est extraite avec de l'Et₂O (5x50 mL). Les phases organiques sont alors rassemblées, séchées sur MgSO₄ et évaporées. Le mélange réactionnel est évaporé à sec et directement purifié sur une colonne de gel de silice greffée C18, en utilisant un gradient d'éluant (H₂O/ACN 95/5 à H₂O/ACN 5/95). Le produit final est obtenu sous forme de poudre blanche avec un rendement de 90% (255 mg; 0,54 mmoles).
**Pf** = 162-164°C; **IR** (KBr) v (cm⁻¹) : 3439, 2935, 1787, 1757, 1728, 1450, 1176, 756; **RMN ¹H** (CDCl₃, 400 MHz): δ 3,65 (s, 3H); 4,65 (s, 2H); 5,79 (s, 2H); 6,86 (d, 2H, *³J* = 8,5 Hz); 6,96 (t, 1H, *³J* = 7,5 Hz); 7,27 (m, 2H); 9,00 (s, 1H); **RMN ¹³C** (CDCl₃, 100 MHz): δ 31,2; 64,8; 66,7; 94,7; 107,7; 114,6 (2C); 121,8; 129,5 (2C); 146,8; 150,0; 153,3; 155,1; 157,5; 162,7; 168,2.

### Exemple 2 : Synthèse d'un type de molecule d'anhydride aza-isatoique selon l'invention doté d'un groupement protecteur thermolabile O- Fluoro phenoxy acetate 13 (correspondant au composé V)

On décrit ici la synthèse d'un autre exemple de composé selon l'invention (selon le schéma 4). Le composé précurseur **6** est fluoro-phénoxyacétylé (un groupement pouvant être plus thermolabile) pour donner le composé **11,** puis iodé pour donner le composé **12** et enfin cyclisé afin d'obtenir le composé **13** aza-isatoique.

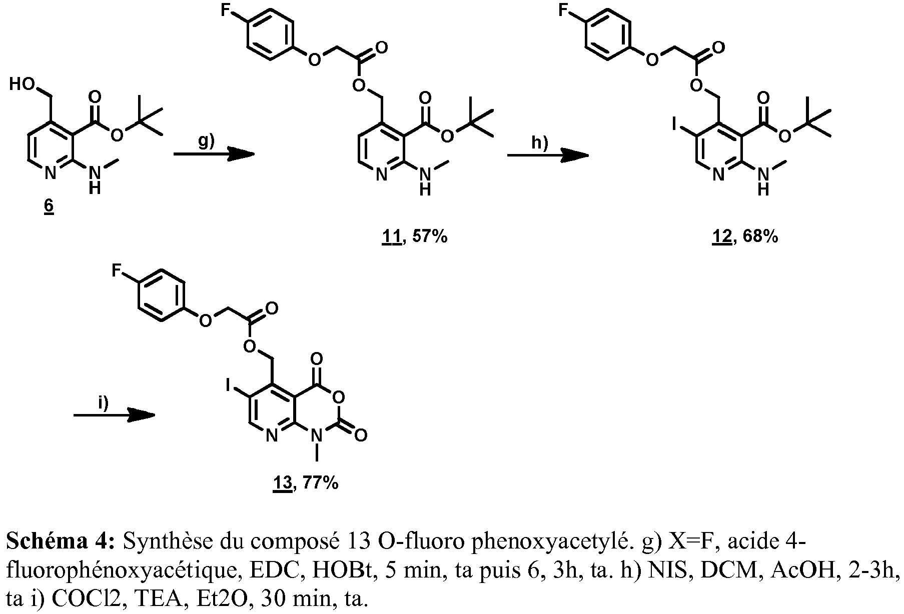

### Exemple 2.1: 4-((2-(4-fluorophénoxy)acétoxy)méthyl)-2-(méthylamino)nicotinate de tert-butyle (11)

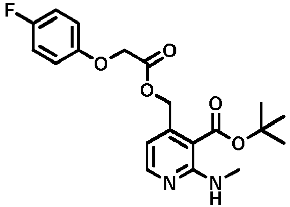

C₂₀H₂₃FN₂O₅

M = 390,40 g/mole

Dans un ballon de 50 mL, sont introduits 149 mg d'acide 4-fluorophénoxyacétique (0,88 mmoles, 1,05 éq), 5 mL de DCM, 169 mg d'EDC (0,88 mmoles, 1,05 éq) et 119 mg d'HOBt (0,88 mmoles, 1,05 éq). Après 5 min à température ambiante, 200 mg de **6** (0,84 mmoles, 1 éq) sont ajoutés et la réaction est laissée sous agitation à température ambiante pendant 3h. 30 mL d'eau sont ensuite ajoutés et la solution est extraite avec de l'Et₂O (3x30 mL). Les phases organiques sont alors rassemblées, séchées sur MgSO₄ et évaporées. Le brut réactionnel est ensuite purifié sur une colonne de gel de silice en utilisant un gradient d'éluant (EP/AcOEt 9/1 à EP/AcOEt 8/2).

Le produit final est obtenu sous forme de poudre jaunâtre avec un rendement de 57% (190 mg, 0.49 mmoles).
Pf = 151-153°C; IR (KBr) v, (cm-1) : 3376, 2977, 2935, 1758 (CO), 1683 (CO), 1586, 1189, 831; RMN 1H (CDCl3, 400 MHz): δ 1,60 (s, 9H); 3,02 (d, 3H, 3J = 4,6 Hz); 4,71 (s, 2H); 5,45 (s, 2H); 6,54 (d, 1H, 3J = 5,2 Hz); 6,88 (m, 2H); 6,99 (t, 2H, 3J = 8,3 Hz); 7,88 (s, 1H); 8,20 (d, 1H, 3J = 5,2 Hz); RMN 13C (CDCl3, 100 MHz): δ 28,1; 28,3 (3C); 65,4; 65,9; 83,1; 105,6; 109,0; 115,9 (d, 3JC-F = 8 Hz, 2C); 116,0 (d, 2JC-F = 23 Hz, 2C); 147,3; 152,0; 153,8 (d, 4JC-F = 2 Hz, 1C); 157,8 (d, 1JC-F = 239 Hz, 1C); 159,5; 167,1; 168,4.

### Exemple 2.2: (6-iodo-1-méthyl-2,4-doxo-2,4-dihydro-1H-pyrido[2,3-d][1,3]oxazine-5-yl)méthyl 2-(4-fluorophénoxy)acétate (12)

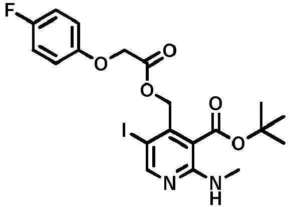

C₂₀H₂₂FIN₂O₅

M = 516,30 g/mole

Dans un ballon de 25 mL, 200 mg de **11** (0,51 mmoles, 1 éq) sont mis en solution dans 3 mL de DCM. 172 mg de NIS (0,77 mmoles, 1,5 éq) et 150 µL d'acide acétique sont ajoutés et le milieu réactionnel est laissé sous agitation pendant 2h. Le mélange réactionnel est ensuite neutralisé avec 20 mL d'une solution aqueuse saturée de thiosulfate de sodium, repris dans 20 mL d'une solution saturée de NaHCO₃, puis extrait avec de l'Et₂O (4×30 mL). Les phases organiques sont alors rassemblées, séchées sur MgSO₄ et évaporées. Le brut réactionnel est ensuite purifié sur une colonne de gel de silice en utilisant un gradient d'éluant (EP à EP/AcOEt 9/1). Le produit final est obtenu sous forme d'huile jaune avec un rendement de 68% (180 mg, 0,35 mmoles).
IR (KBr): v, 3413, 2926, 1763, 1688, 1506, 1183, 828; RMN 1H (CDCl3, 400 MHz): δ 1,56 (s, 9H); 2,97 (d, 3H, 3J = 4,64 Hz); 4,60 (s, 2H); 5,37 (s, 2H); 6,85 (m, 2H); 6,96 (m, 2H); 8,50 (s, 1H); RMN 13C (CDCl3, 100 MHz): δ 28,1 (3C); 28,4; 65,7; 68,7; 82,4; 83,7; 112,7; 115,9 (d, 2JC-F = 23 Hz, 2C); 116,0 (d, 3JC-F = 8 Hz, 2C); 144,5; 153,7; 157,7; 157,8 (d, 1JC-F = 239 Hz, 1C); 158,3; 166,4; 168,2.

### Exemple 2.3: (6-iodo-1-méthyl-2,4-doxo-2,4-dihydro-1H-pyrido[2,3-d][1,3]oxazine-5-yl)méthyl 2-(4-fluorophenoxy)acétate (13)

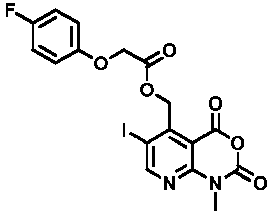

C₁₇H₁₂FIN₂O₆

M = 486,19 g/mole

Dans un ballon de 50 mL sous atmosphère d'azote, 160 mg de **12** (0,31 mmoles, 1 éq) sont mis en solution dans 3 mL de DCM. 489 µL de phosgène (20% dans le THF, 0,98 mmoles, 9 éq) et 129 µL de TEA (0,98 mmoles, 9 éq) sont ajoutés simultanément et la réaction est laissée sous agitation pendant 30 min. 30 mL d'eau sont ensuite ajoutés et la solution est extraite avec de l'Et₂O (3x30 mL). Les phases organiques sont alors rassemblées, séchées sur MgSO₄ et évaporées. Le mélange réactionnel est évaporé à sec et directement purifié sur une colonne de gel de silice greffée C18, en utilisant un gradient d'éluant (H₂O/ACN 95/5 à H₂O/ACN 5/95).

Le produit final est obtenu sous forme de poudre blanche avec un rendement de 77% (115 mg; 0,24 mmoles).
Pf = 150-152°C; IR (KBr) v (cm-1) : 3454, 3078, 2934, 1787, 1745, 1504, 1194, 825; RMN 1H (CDCl3, 400 MHz): δ 3,66 (s, 3H); 4,62 (s, 2H); 5,78 (s, 2H); 6,83 (m, 2H); 6,95 (m, 2H); 9,01 (s, 1H); RMN 13C (CDCl3, 100 MHz): δ 31,2; 65,6; 66,8; 94,7; 107,7; 115,9 (d, 2JC-F = 23 Hz, 2C); 115,9 (d, 3JC-F = 8 Hz, 2C); 146,7; 150,0; 153,4; 153,7 (d, 4JC-F = 2 Hz, 1C); 155,2; 157,8 (d, 1JC-F = 239 Hz, 1C); 162,8; 168,1.

### Exemple 3 : Synthèse d'un type de molecule d'anhydride aza-isatoique selon l'invention doté d'un groupement protecteur acido/thermolabile N-Boc 18 (correspondant au composé VI)

On décrit ici la synthèse d'un autre exemple de composé selon l'invention (selon le schéma 5). Le composé précurseur **6** est tout d'abord transformé en composé azido **15** précurseur d'une composé aminé sur laquelle fonction amine sera couplée le groupement protecteur BOC pour accéder au composé **16.** Une réaction d'iodation donne le composé **17** et enfin de cyclisation permet d'accéder au composé **18** aza-isatoique.

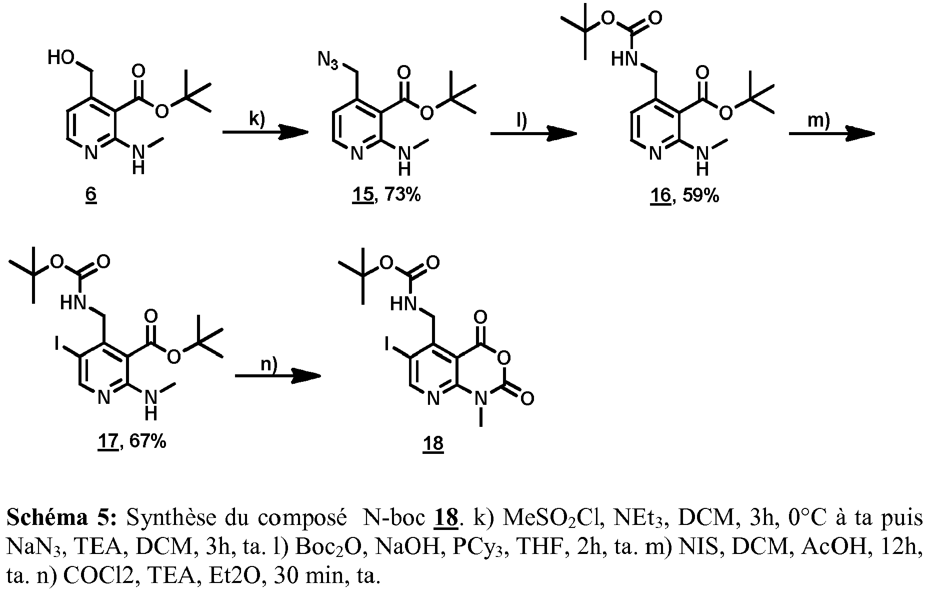

### Exemple 3: 4-(azidométhyl)-2-(méthylamino)nicotinate de tert-butyle (15)

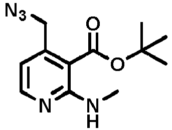

C₁₂H₁₇N₅O₂

M = 263,30 g/mole

Dans un ballon de 100 mL sous azote, 1.40 g de **6** (5,88 mmoles, 1 éq) sont mis en solution dans 20 mL de DMF. A 0°C, 0,91 mL de MeSO₂Cl (11.76 mmoles, 2 éq) et 4,08 mL de TEA (29.3 mmol, 5 éq) sont ajoutés simultanément. Le mélange réactionnel est laissé sous agitation à température ambiante et le suivi est réalisé par CCM (EP/Et₂O 1/1). Après formation complète du dérivé mésylate correspondant, 1.15 g de NaN₃ (17,64 mmoles, 3 éq) sont ajoutés et le mélange est laissé sous agitation pendant 3h à température ambiante. 50 mL d'eau sont ensuite ajoutés et la solution est extraite avec de l'Et₂O (3x60 mL). Les phases organiques sont alors rassemblées, séchées sur MgSO₄ et évaporées. Le brut réactionnel est ensuite purifié sur une colonne de gel de silice en utilisant un gradient d'éluant (EP/ Et₂O 9/1 à EP/ Et₂O 8/2). Le produit final est obtenu sous forme d'huile jaune avec un rendement de 73% (1,13 g, 4,29 mmoles).
IR (KBr): v, 3374, 2978, 2104 (N3), 1679, 1586, 1425, 1125, 847, 655; RMN 1H (CDCl3, 400 MHz): δ 1,60 (s, 9H); 3,01 (d, 3H, 3J = 5,0 Hz); 4,58 (s, 2H); 6,61 (d, 1H, 3J = 5,0 Hz); 7,73 (s, 1H); 8,24 (d, 1H, 3J = 5,0 Hz); RMN 13C (CDCl3, 100 MHz): δ 28,3 (3C); 53,9; 77,0; 83,1; 106,7; 111,5; 146,8; 152,0; 159,5; 167,1.

### Exemple 3.1 : 4-(((tert-butoxycarbonyl)amino)méthyl)-2-(méthylamino)nicotinate de tert-butyle (16)

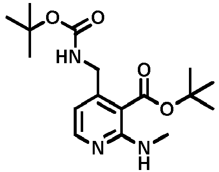

C₁₇H₂₇N₃O₄

M = 337,41 g/mole

Dans un ballon de 25 mL, 180 mg de **15** (0,68 mmoles, 1 éq) et 447 mg de Boc₂O (2,05 mmoles, 3 éq) sont mis en solution dans 5 mL de THF. 750 µL d'une solution aqueuse de NaOH (0,75 mmoles, 1,1 éq, 1M dans H₂O) et 230 mg de PCy3 sont ajoutés successivement. La réaction est laissée sous agitation à température ambiante pendant 2h. 30 mL d'eau sont ensuite ajoutés et la solution est extraite avec de l'Et₂O (3x30 mL). Les phases organiques sont alors rassemblées, séchées sur MgSO₄ et évaporées. Le brut réactionnel est ensuite purifié sur une colonne de gel de silice en utilisant un gradient d'éluant (EP/ AcOEt 9/1 à EP/ AcOEt 8/2). Le produit final est obtenu sous forme d'huile jaune avec un rendement de 59% (135 mg, 0,40 mmoles).
RMN 1H (CDCl3, 400 MHz): δ 1,44 (s, 9H); 1,60 (s, 9H); 3,01 (d, 3H, 3J = 4,6 Hz); 4,40 (d, 2H, 3J = 6,1 Hz); 5,02 (sl, 1H); 6,57 (d, 1H, 3J = 5,1 Hz); 7,52 (sl, 1H); 8,18 (d, 1H, 3J = 5,1 Hz); RMN 13C (CDCl3, 100 MHz): δ 28,4 (6C); 28,5; 43,9; 79,6; 83,0; 107,6; 111,6; 150,5; 151,5; 155,7; 159,2; 167,4.

### Exemple 3.2 : 4-(((tert-butoxycarbonyl)amino)méthyl)-5-iodo-2-(méthylamino)nicotinate de tert-butyl (17)

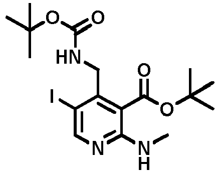

C₁₇H₂₆IN₃O₄

M = 463,31 g/mole

Dans un ballon de 25 mL, 130 mg de **16** (0,39 mmoles, 1 éq) sont mis en solution dans 4 mL de DCM. 225 mg de NIS (0,58 mmoles, 1,5 éq) et 200 µL d'acide acétique sont ajoutés et le milieu réactionnel est laissé sous agitation pendant 12h. Le mélange réactionnel est ensuite neutralisé avec 10 mL d'une solution aqueuse saturée de thiosulfate de sodium, repris dans 10 mL d'une solution saturée de NaHCO₃, puis extrait avec de l'Et₂O (4×30 mL). Les phases organiques sont alors rassemblées, séchées sur MgSO₄ et évaporées. Le brut réactionnel est ensuite purifié sur une colonne de gel de silice en utilisant un gradient d'éluant (EP à EP/AcOEt 9/1).

Le produit final est obtenu sous forme de poudre jaune avec un rendement de 67% (121 mg, 0,26 mmoles).
RMN 1H (CDCl3, 400 MHz): δ 1,44 (s, 9H); 1,60 (s, 9H); 2,95 (d, 3H, 3J = 4,6 Hz); 4,39 (d, 2H, 3J = 5,2 Hz); 4,82 (sl, 1H); 6,67 (sl, 1H); 8,48 (s, 1H); RMN 13C (CDCl3, 100 MHz): δ 28,1 (3C); 28,3 (4C); 47,6; 79,5; 82,9; 83,9; 113,1; 147,7; 154,9; 157,5; 158,3; 166,7.

### Exemple 3.4 : (6-iodo-1-méthyl-2,4-doxo-2,4-dihydro-1H-pyrido[2,3-d][1,3]oxazine-5-yl) méthyl carbamate de tert-butyle (18)

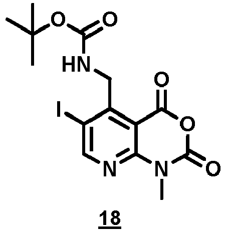

Dans un ballon de 25 mL sous atmosphère d'azote, 100 mg de 17 (0,22 mmoles, 1 éq) sont mis en solution dans 3 mL de DCM. 342 µL de phosgène (0,65 mmoles, 20% dans le toluène, 3 éq) et 90 µL de TEA (0,65 mmoles, 3 éq) sont ajoutés simultanément et la réaction est laissée sous agitation à température ambiante pendant 30 min. Cette opération est répétée à trois reprises pour convertir la totalité de la matière première. 30 mL d'eau sont ensuite ajoutés et la solution est extraite avec du DCM (3x30 mL). Les phases organiques sont alors rassemblées, séchées sur MgSO₄ et évaporées. L'anhydride azaisatoïque **18** ainsi obtenu a été directement mis en réaction sans étape de purification supplémentaire.

### Exemple 4: Réaction des molécules dérivées de l'anhydride aza-isatoique selon l'invention avec un composé aminé

Cet exemple démontre que les molécules synthétisées dans les exemples 1 à 3, soit respectivement les molécules **9, 13,** et **18** réagissent avec un composé aminé tel que la phényl éthyl amine.

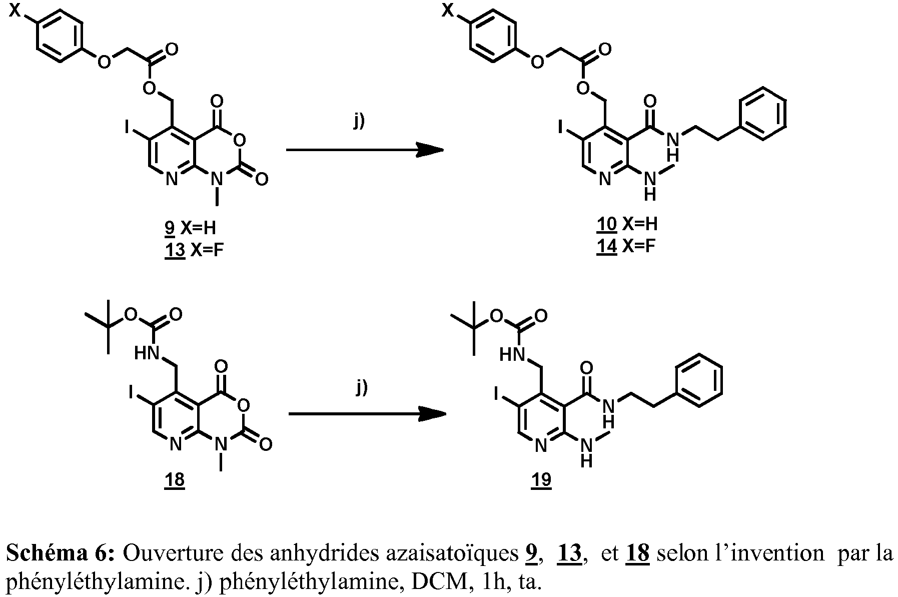

### Exemple 4.1 : (5-iodo-2-(méthylamino)-3-(phénéthylcarbamoyl)pyridine-4-yl)méthyl 2-phénoxyacétate (10)

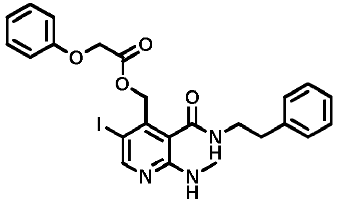

C₂₄H₂₄IN₃O₄

M = 545,37 g/mole

Dans un ballon de 10 mL, sont introduits 65 mg de 9 (0,13 mmoles, 1 éq), 1 mL de DCM et 17,4 µL de phényléthylamine (0,13 mmoles, 1 éq). Le mélange réactionnel est laissé sous agitation pendant 1h à température ambiante. 15 mL d'eau sont ensuite ajoutés et la solution est extraite avec de l'Et₂O (5x20 mL). Les phases organiques sont alors rassemblées, séchées sur MgSO₄ et évaporées. Le brut réactionnel est ensuite purifié sur une colonne de gel de silice en utilisant un gradient d'éluant (EP/AcOEt 9/1 à EP/AcOEt 7/3).

Le produit final est obtenu sous forme de poudre jaunâtre avec un rendement de 71% (50 mg, 0,09 mmoles).
Pf = 164-166°C; IR (KBr) v (cm-1) : 3428, 3296, 2924, 1761, 1627, 1433, 1170, 754; RMN 1H (CDCl3, 400 MHz): δ 2,86 (s, 3H); 2,88 (d, 2H, 3J = 7,1 Hz); 3,69 (m, 2H); 4,57 (s, 2H); 4,98 (s, 2H); 5,58 (d, 1H, 3J = 4,5 Hz); 6,54 (t, 1H, 3J = 5,6 Hz); 6,88 (d, 2H, 3J = 8,8 Hz); 7,00 (t, 1H, 3J = 7,6 Hz); 7,21 (m, 2H); 7,28 (m, 5H); 8,40 (s, 1H); RMN 13C (CDCl3, 100 MHz): δ 28,4; 35,2; 40,6; 64,9; 67,3; 80,3; 114,6 (2C); 118,6; 122,0; 126,8; 128,6 (2C); 128,7 (2C); 129,7 (2C); 138,1; 140,7; 156,3; 156,8; 157,5; 166,5; 168,3.

### Exemple 4.2 : (5-iodo-2-(méthylamino)-3-(phénéthylcarbamoyl)pyridine-4-yl)méthyl 2-(4-fluorophénoxy)acétate (14)

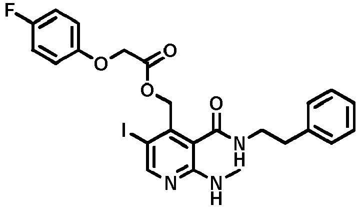

C₂₄H₂₃FIN₃O₄

M = 563,36 g/mole

Dans un ballon de 10 mL, sont introduits 50 mg de **13** (0,10 mmoles, 1 éq), 1 mL de DCM et 12,9 µL de phényléthylamine (0,10 mmoles, 1 éq). La réaction est laissée sous agitation à température ambiante pendant 1h. 10 mL d'eau sont ensuite ajoutés et la solution est extraite avec de l'Et₂O (5x15 mL). Les phases organiques sont alors rassemblées, séchées sur MgSO₄ et évaporées. Le brut réactionnel est ensuite purifié sur une colonne de gel de silice en utilisant un gradient d'éluant (EP/AcOEt 7/3 à EP/AcOEt 6/4). Le produit final est obtenu sous forme de poudre jaunâtre avec un rendement de 87% (50 mg, 0,09 mmoles).
Pf = 165-167°C; IR (KBr): v, 3388, 3376, 2924, 1742, 1661, 1577, 1504, 1191, 824; RMN 1H (CDCl3, 400 MHz): δ 2,86 (d, 3H, 3J = 4,64 Hz); 2,89 (t, 2H, 3J = 6,84 Hz); 3,72 (m, 2H); 4,51 (s, 2H); 4,99 (s, 2H); 5,55 (d, 1H, 3J = 4,6 Hz); 6,51 (t, 1H, 3J = 8,0 Hz); 6,83 (m, 2H); 6,97 (m, 2H); 7,21 (m, 3H); 7,30 (m, 2H); 8,40 (s, 1H); RMN 13C (CDCl3, 100 MHz): δ 28,4; 35,1; 40,5; 65,6; 67,3; 80,2; 115,9 (d, 3JC-F = 8 Hz, 2C); 116,1 (d, 2JC-F = 23 Hz, 2C); 118,6; 126,8; 128,6 (2C); 128,7 (2C); 138,1; 140,7; 153,6 (d, 4JC-F = 2 Hz, 1C); 156,3; 156,8; 157,9 (d, 1JC-F = 222 Hz, 1C); 166,5; 168,1.

### Exemple 4.3: ((5-iodo-2-(méthylamino)-3-(phénéthylcarbamoyl)pyridine-4-yl)méthyl)carbamate de tert-butyle (19)

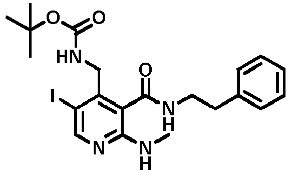

C₂₁H₂₇IN₄O₃

M = 510,37 g/mole

Dans un ballon de 10 mL, l'anhydride azaisatoïque **18** est mis en solution dans 1 mL de DCM. 27,6 µL de phényléthylamine (0,22 mmoles, 1 éq) sont alors ajoutés et le milieu réactionnel est laissé sous agitation à température ambiante pendant 1h. 10 mL d'eau sont ensuite ajoutés et la solution est extraite avec de l'Et₂O (4x20 mL). Les phases organiques sont alors rassemblées, séchées sur MgSO₄ et évaporées. Le brut réactionnel est ensuite purifié sur une colonne de gel de silice en utilisant un gradient d'éluant (EP/AcOEt 7/3 à EP/AcOEt 4/6).

Le produit final est obtenu sous forme de poudre jaunâtre avec un rendement de 54% (61 mg, 0,12 mmoles).
RMN 1H (CDCl3, 400 MHz): δ 1,34 (s, 9H); 2,79 (d, 3H, 3J = 4,6 Hz); 2,88 (t, 2H, 3J = 7,1 Hz); 3,67 (m, 2H); 3,87 (d, 2H, 3J = 6,5 Hz); 5,47 (s1, 1H); 5,72 (s1, 1H); 7,18 (m, 5H); 8,27 (s, 1H); 8,98 (s1, 1H); RMN 13C (CDCl3, 100 MHz): δ 28,3 (3C); 28,4; 30,0; 40,6; 44,9; 80,0; 80,6; 118,9; 126,4; 128,4 (2C); 128,7 (2C); 138,8, 143,1; 156,1; 156,2; 156,7; 166,7.

### Exemple 5: Procédé de déprotection des composés aza-anthranylates et libération de la phényléthyl amine en conditions de PCR « hot-start »

Nous démontrons ici que les dérivés **10, 14,** et **19** synthétisés à l'exemple 3 peuvent se cliver dans des conditions de PCR « hot-start » et libérer la phényléthyle amine qui mime une protéine.

### Procédure générale:

Dans un tube de 1,5 mL, sont introduits : 20 µL d'une solution d'amide (**10**, **14** ou **19**) à 2,5 mM dans le DMSO et 180 µL d'un tampon utilisé de façon classique dans une réaction d'amplification de matériel génétique (60 mM Tris pH 9, KC1 50 mM, MgCl₂ 1 mM) . Le mélange est alors laissé sous agitation à 95°C dans un thermomixer. Des suivis LCMS (condition 1) ont été réalisés à t = 15 min, 30 min et 1h.

### Exemple 5.1: Evaluation du clivage à 95°C du dérivé aza-anthranylate phénoxyacétate 10

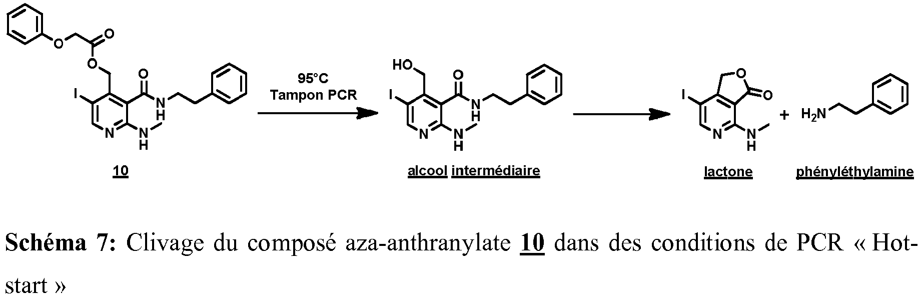

La déprotection thermique du groupement phénoxyacétate conduit à la formation de l'alcool benzylique correspondant (cf Schéma 7). L'attaque nucléophile de l'alcool sur le carbonyle mis en jeu dans la liaison amide permet alors de relarguer la phényléthylamine dans le milieu en générant la lactone correspondante.

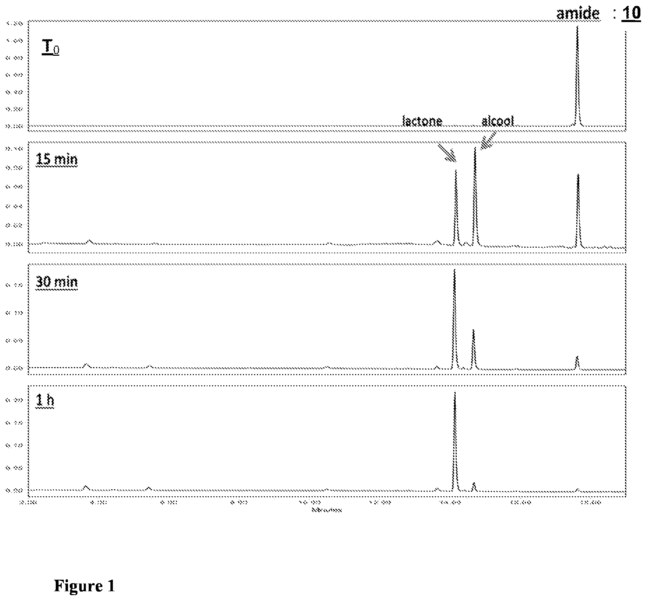

Cette cascade réactionnelle est représentée sur les chromatogrammes HPLC de la Figure 1.

Après 15 minutes à 95°C, on remarque la formation de l'alcool déprotégé et de la lactone attendue ce qui indique la libération de phényléthylamine. Ce résultat montre bien le caractère thermolabile du groupement phénoxyacétate conduisant à la formation de l'alcool correspondant. Après cyclisation, on retrouve la présence de la lactone attendue témoignant du clivage de la liaison amide et donc du relargage de la phényéthylamine dans le milieu. Après 1h à 95°C, la population d'amide de départ et d'alcool ont quasiment totalement disparu au profit de la lactone.

Ce résultat démontre la possibilité de cliver une liaison amide à 95°C par ce système de cyclisation intramoléculaire.

### Exemple 5.2: Evaluation du clivage à 95°C du dérivé aza-anthranylate fluoro phénoxyacétate 14

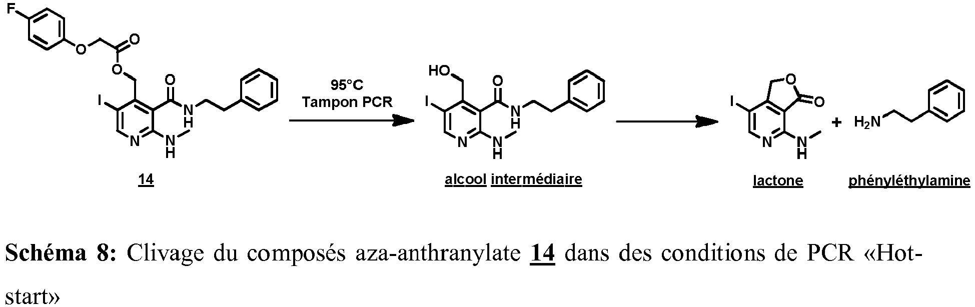

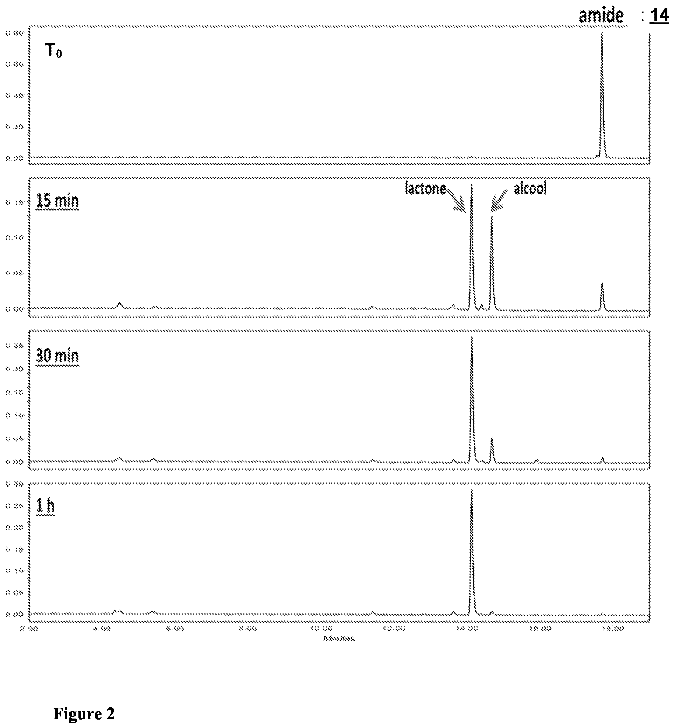

Le procédé de déprotection a également été mis en œuvre à partir du dérivé fluorophenoxyacétate **14.** Après 15 minutes à 95°C, la population d'amide est très faible. En particulier, elle est plus faible par comparaison au résultat observé avec le dérivé **10** (voir Figure 2). La présence de l'atome de fluor accélère le clivage du groupement phénoxyacétate. Ainsi, après 30 minutes, cette population d'amide a quasiment totalement disparu au profit de l'alcool et de la lactone très majoritaire indiquant la libération de phényléthylamine.

### Exemple 5.3 : Evaluation du clivage à 95°C du dérivé aza-anthranylate N-Boc 19

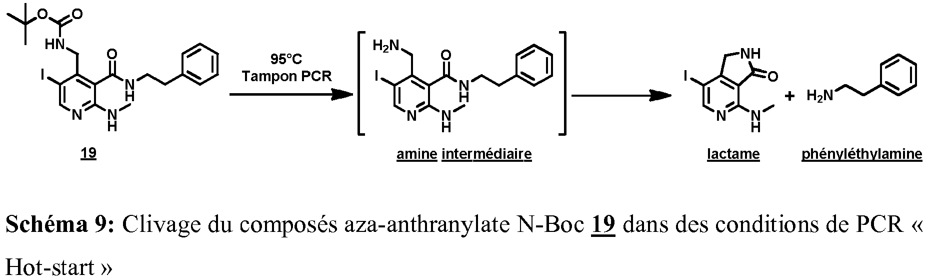

Dans ce cas, la déprotection du groupement N-boc conduit au dérivé amine benzylique intermédiaire.

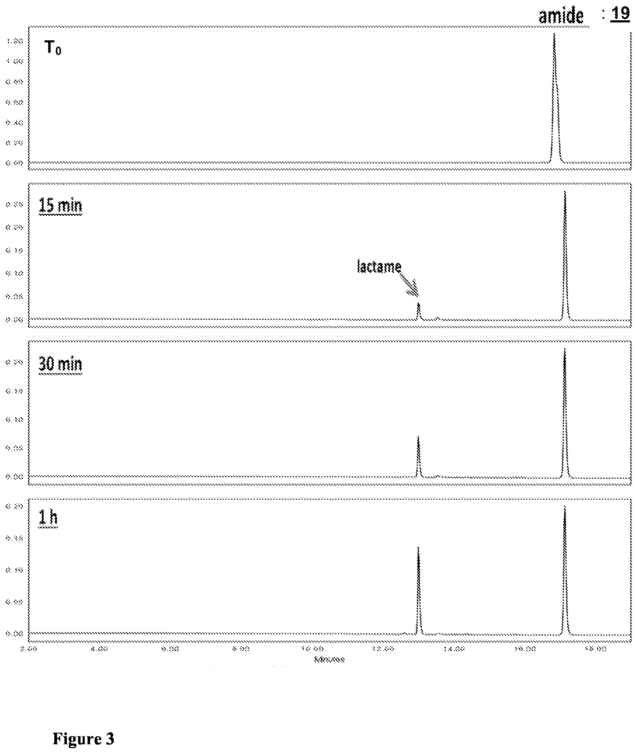

Ce dernier peut alors cycliser pour former le lactame correspondant en libérant la phényléthylamine dans le milieu (Schéma 9 ci-dessus). Le suivi par HPLC montrant les différents composés intermédiaires est représenté sur la Figure 3.

Pour ce dérivé, la forme déprotégée non cyclisée (amine intermédiaire) n'est pas détectée. Cet exemple démontre donc la cyclisation immédiate après déprotection du groupement N-boc pour former la phényléthylamine. Ceci confirme l'avantage de la fonction amino en position 5 par rapport à la fonction alcool, pour la vitesse de cyclisation. L'homme du métier saura donc trouver le groupement protecteur approprié de la fonction NH₂ en position 5 du cycle aromatique pour obtenir une déprotection suffisamment rapide conduisant à une cyclisation instantanée, et à la libération de la phényléthylamine.

### Exemple 6: Protection réversible de l'hémoglobine par le composé aza-isatoique 13 tel que décrit dans l'invention

Nous démontrons ici que le dérivé aza-isatoique **13** doté du groupement thermolabile fluoro-phenoxy est capable de réagir avec une protéine dans des conditions douces et en milieu aqueux pour former un dérivé protéine-aza anthranylate fluoro-phenoxy.

L'élimination de ce groupement protecteur suite à un traitement thermique à 95°C permet de régénérer la protéine native.

### Protocole:

On réalise les mélanges tels que décrits ci-dessous où les expériences AL600 1x à AL 600 0.25X correspondent respectivement à 33µg d'hémoglobine mis en réaction avec des concentrations croissantes du composé aza-isatoïque dans un mélange de DMSO et de PBS à pH 7.4 (PBS = Phosphate buffered Saline issu de la dissolution d'une pastille de la référence Sigma P4417 dans 200 ml d'eau (pH 7,4)). Les mélanges sont mis à incuber à température ambiante pendant 2 heures avant de prélever un aliquot pour analyse par HPLC sur une colonne Waters (Milford, USA) XBridge BEH 300 C4 avec un gradient de 20 à 72% d'acétonitrile dans une solution d'acide trifluoroacetique à 10 mM en 120 min.

**Tableau 1: Concentration relative en réactifs pour réaliser l'acylation réversible de l'hémoglobine**

| | Hémoglobine Humaine (Sigma H7379) 6.6µg/µl dans le PBS | PBS pH 7,4 | DMSO | **13** (41 mM dans le DMSO) |
|---|---|---|---|---|
| EXPERIENCES | µl | µl | µl | µl |
| AL 600 0.25x | 5 | 5 | 8,75 | 1,25 |
| AL 600 0.5x | 5 | 5 | 7,5 | 2,5 |
| AL 600 1x | 5 | 5 | 5 | 5 |

| CTRLES SANS Réactif acylant | | | | |
|---|---|---|---|---|
| AL 602 CTRLE 0.25X | 5 | 5 | 10 | 0 |

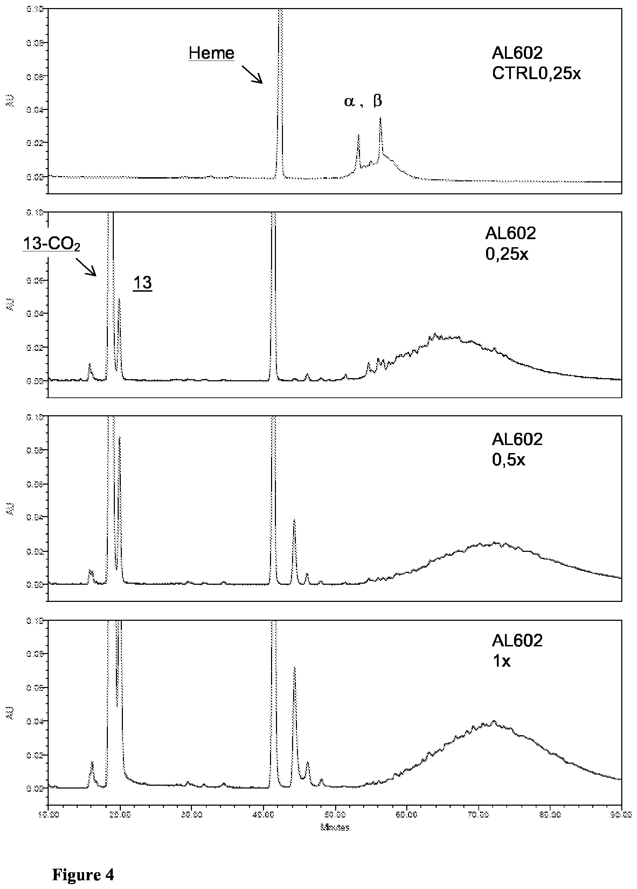

Sur la Figure 4, le chromatogramme AL602 CTRLE 0.25X montre l'hémoglobine de contrôle n'ayant pas réagi avec le composé **13**. On y visualise l'hème et les 2 sous unités alpha et beta de la partie protéique. Les 3 chromatogrammes suivants (AL 600 0.25 X-1X) montrent la disparition de la partie protéique au profit d'un massif décalé vers la droite correspondant aux sous unités alpha et béta acylé par le composé aza-isatoique **13.** L'élargissement du pic correspond à l'acylation aléatoire des sites réactifs de la protéine.

On démontre ainsi que le composé aza-isatoique tel que décrit selon l'invention peut réagir avec une protéine.

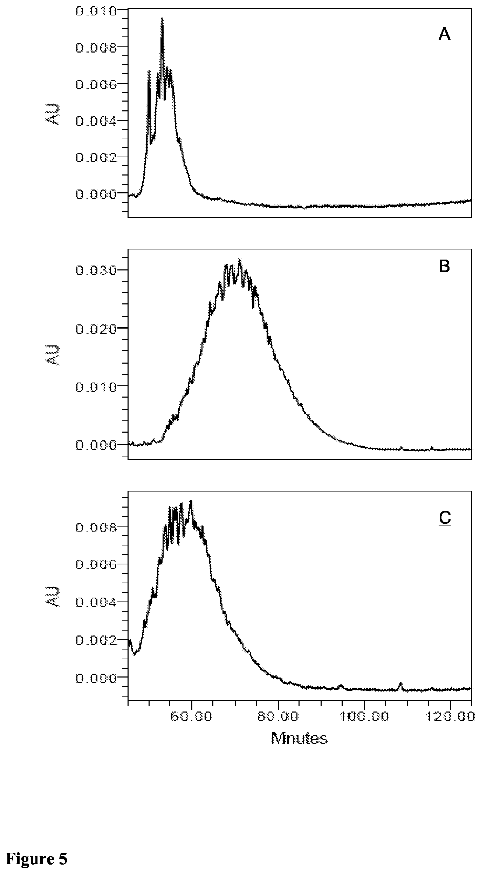

Lorsque le milieu réactionnel AL 600 1X est soumis à un traitement thermique à 95°C pendant 15 min dans un tampon de PCR (constitué pour partie principale de Tris pH9), on observe une hydrolyse de la partie benzylamide qui conduit à restaurer le profil chromatographique de l'hémoglobine native. Ceci est visible sur la Figure 5 où le chromatogramme du haut (A) représente l'hémoglobine native et reprise dans du GuHCl 8M, le chromatogramme du milieu (B) représente l'hémoglobine acylée par le composé **13** et reprise dans du GuHCl 8M et, où le chromatogramme du bas (C) représente l'hémoglobine acylée par le composé **13** puis chauffée dans du tampon Tris pH8 et reprise dans du GuHCl 8M. On voit distinctement comme précédemment que l'acylation de l'hémoglobine conduit à la formation d'un massif de protéine acylé puis que ce massif disparaît suite à un traitement thermique pour régénérer la protéine native.

A noter que les milieux réactionnels ont été repris après réaction dans du GuHCl 8M afin de solubiliser la partie protéique qui aurait précipité lors du traitement thermique. Cet exemple démontre l'acylation réversible d'une protéine modèle par le composé **13** tel que décrit dans l'invention.

### Exemple 7: Démonstration de l'acylation reversible de la TAQ polymerase par le composé aza-isatoique 13 tel que décrit dans l'invention

Nous démontrons ici que le dérivé aza-isatoique **13** doté du groupement thermolabile fluoro-phenoxy est capable de réagir avec une polymerase thermostable (*Taq*) dans des conditions douces et en milieu aqueux pour former un dérivé *Taq-aza* anthranylate fluorophenoxy. L'élimination de ce groupement protecteur suite à un traitement thermique à 95°C dans des conditions de PCR permet de restaurer l'activité de la polymérase, ce qui est démontré par le dosage de son activité.

Le concept d'utiliser des anhydrides aza-isatoique convenablement modifiés pour masquer transitoirement l'activité d'une polymérase et la restaurer ensuite après un traitement thermique est ainsi démontré.

### Protocole :

Nous avons utilisé la *Taq* Genscript (2500 u /100µl REF E00012), mais afin d'éliminer toutes traces de nucléophiles dans cette enzyme nous avons effectué préalablement un changement de tampon par passage sur un microcon 10 KD Amicon (n° 42407). La suspension enzymatique est ainsi déposée sur le microcon et centrifugée jusqu'à épuisement du tampon. Cinq lavages sont effectués avec 100 µl de PBS pH 7,4, puis le dernier retentât est récupéré par inversion du tube et complété à QSP 20µl avec du PBS pour obtenir une suspension à 125 n/µl. Nous avons ainsi démontré que le Tris et le glycérol était éliminé de façon très satisfaisante par cette méthode.

On réalise ensuite les mélanges tels que décrit dans le Tableau **2** où les expériences AL604 0.25x à AL 604 0.375X correspondent respectivement à 625 unités de *Taq* polymerase mis en réaction avec des concentrations croissantes du composé aza-isatoïque dans un mélange de DMSO et de PBS à pH 7.4 (PBS = Phosphate buffered Saline issu de la dissolution d'une pastille de la référence Sigma P4417 dans 200 ml d'eau (pH 7,4)). Les mélanges sont mis à incuber à température ambiante pendant 3 heures sous vortex léger avant d'évaluer leur activité enzymatique dans des conditions de PCR « hot-start ».

**Tableau 2: Concentration relative en réactifs pour réaliser l'acylation réversible de la TAQ polymérase**

| | *Taq* Genscript (125 u/µl) | PBS pH 7,4 | DMSO | **13** (41 mM dans le DMSO) | Concentration en TAQ] finale (20 µl à 50/50 PBS/DMSO) |
|---|---|---|---|---|---|
| EXPERIENCES | Unités/µl | µl | µl | µl | u/µl |
| AL 604 0.25x | 625 u/5 µl | 5 | 8,75 | 1,25 | 31 |
| AL 604 0,375x | 625 u/5 µl | 5 | 8,1 | 1,9 | 31 |

### Dosage de l'activité polymérase de la TAQ modifiée par le composé 13:

L'activité de polymérisation de la TAQ polymérase est réalisée à l'aide d'une sonde oligonucléotidique de 45 bases terminée par une structure en « épingle à cheveux ». Celle-ci est caractérisée par la présence d'un quencher de fluorescence en début de structure et par un fluorophore en fin de séquence de la sonde, de telle manière à ce que le quencher se retrouve spatialement proche du fluorophore et qu'aucun signal de fluorescence ne puisse être mesuré dans cette configuration. Sous l'action de l'activité polymérase, cette sonde est élonguée à l'aide d'un oligonucléotide de 19 bases complémentaire au début de la sonde précédente. Sous l'action de l'élongation, la structure en épingle à cheveux de la sonde se déplie et le fluorophore peut émettre et une fluorescence est alors mesurable. Cette mesure de fluorescence est effectuée à une température de 60°C pendant 20 minutes en présence des réactifs nécessaires à l'activité de l'enzyme, c'est-à-dire des dNTP, du MgCl2 et un tampon basique à pH 9.5.

L'augmentation de la fluorescence est linéaire en début de mesure et permet de calculer une vitesse initiale correspondant à la quantité de fluorescence émise par minutes d'élongation. En mesurant cette vitesse initiale pour différentes concentrations d'une polymérase donnée ayant une activité connue en U/µL, il est possible d'établir une droite étalon permettant de mesurer l'activité d'une enzyme similaire et d'activité inconnue.

La modification chimique de la polymérase afin de la rendre inactive est mesurée par cette méthode. En mesurant le niveau d'activité résiduelle après modification de l'enzyme, il est possible de déterminer l'efficacité de la protection mise en place. Une polymérase totalement inactivée par modification chimique ne doit plus générer d'activité sans activation thermique à des températures supérieures à 90°C.

La capacité à restaurer l'activité de l'enzyme après chauffage de 15 min à 95°C par exemple est facilement mesurable par la même méthode.

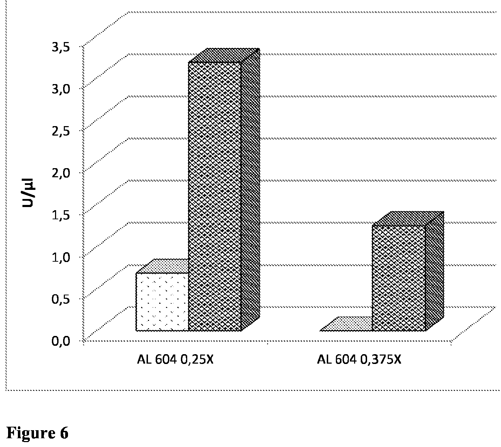

La Figure 6 démontre donc qu'en fonction de la concentration choisie en agent acylant, on peut inactiver totalement l'activé polymérase de la *Taq* et la restaurer suite à un traitement à 95°C pendant 15 min dans des conditions de « Hot-start » PCR (voir colonne correspondant à AL 604 0,375X).

## Revendications

1. Composé de formule (I) suivante : 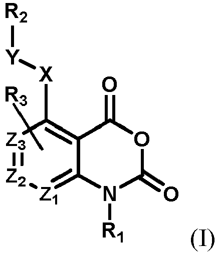 dans laquelle
X est une liaison covalente ou un alkyle en C₁ à C₄,
Y est un radical nucléophile choisi parmi O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-NH-O, O-C(O)-O-NR₄, NH-C(O)-O-NR₄, C(O)-S, et R₄ est H ou un groupe alkyle en C₁ à C₄,
Z₁, Z₂, Z₃ représentent chacun indépendamment l'un de l'autre, N ou C, de préférence Z₃ représente C, plus préférentiellement Z₃ est C et R₃ est en position Z₃,
R₁ est H, un groupe alkyle en C₁ à C₆, un alcényle, un groupe aryle, ou un hétérocycle, lesdits groupes alkyle, alcényle, aryle, ou hétérocycle, étant éventuellement substitués par un ou plusieurs groupes fonctionnels choisi parmi les groupes amine, imine, nitrile, cyano, amide, imide, hydroxyle, alcoxyle, carbonyle, carboxyle, ester, thiol, thioéther, thioester et halogénure, de préférence R₁ est un groupe méthyle ou éthyle,
R₂ est un groupement protecteur thermolabile, c'est à dire un groupement protecteur qui est stable à température entre 15°C et 25°C, et qui est clivé, dissocié ou relargué d'une molécule à laquelle il est lié, par un traitement thermique à une température comprise entre 50 et 100°C ; et/ou R₂ est un groupement protecteur acidolabile, c'est-à-dire un groupement protecteur qui est stable en condition neutre ou basique et qui est clivé, dissocié ou relargué, par traitement à un pH inférieur à 6,0,
R₃ est H, un groupe alkyle en C₁ à C₁₂, par exemple un groupe iso-propyl, isobutyl, sec-butyl, tert-butyl, isopentyl ou 2,2-diméthylpropyl, un groupe aryle, un hétérocycle, un groupe acyle, un groupe alcényle, un halogène (e.g. F, Cl, Br et I), ou un groupe cyano, lesdits groupes alkyle, , aryle, hétérocycle, ou alcényle, étant éventuellement substitués par un ou plusieurs groupes fonctionnels choisi parmi les groupes amine, imine, nitrile, cyano, amide, imide, hydroxyle, alcoxyle, carbonyle, carboxyle, ester, thiol, thioéther, thioester et halogénure,
et lorsque Y est choisi parmi O, S et NR₄, X n'est pas une liaison.

2. Composé selon la revendication 1 de formule (II) suivante :

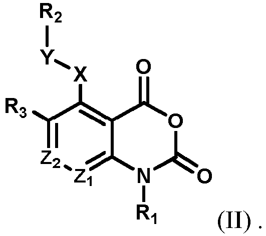

3. Composé selon l'une des revendications 1 ou 2, **caractérisé en ce que** le groupement thermolabile et/ou acidolabile R₂ est choisi parmi les groupements *tert*-butoxycarbonyl (BOC), phénoxyacétyle substitué ou non, trityle, méthoxytrityle, diméthoxytrityle ou citraconyle.

4. Composé selon l'une quelconque des revendications précédentes, **caractérisé en ce que** R₁ est un groupe méthyle.

5. Composé selon l'une quelconque des revendications précédentes, **caractérisé en ce que** R₃ est l'iode.

6. Composé selon l'une quelconque des revendications précédentes, **caractérisé en ce que** Z₁ est N et Z₂ est C.

7. Composé selon l'une quelconque des revendications précédentes **caractérisé par** l'une des structures suivantes :

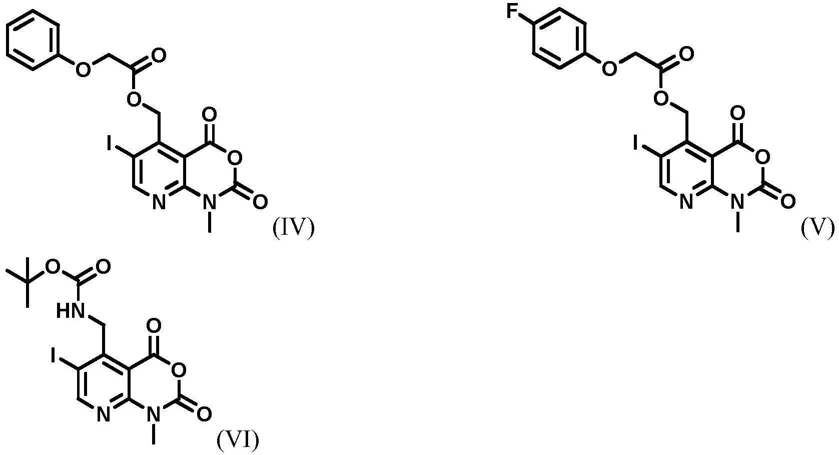

8. Procédé de préparation d'une molécule biologique protégée, comprenant la mise en contact d'un composé selon l'une des revendications 1 à 7 avec une molécule biologique comprenant un ou plusieurs groupes nucléophiles, dans des conditions permettant l'acylation d'un ou plusieurs groupes nucléophiles de ladite molécule biologique, pour former une molécule biologique protégée.

9. Molécule biologique protégée, susceptible d'être obtenue par le procédé de la revendication 8.

10. Molécule biologique protégée, de formule (III) suivante : 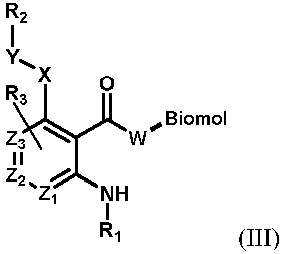 dans laquelle
Biomol est une molécule biologique
W est un groupe nucléophile de la molécule biologique, par exemple NH, S ou O,
X est une liaison covalente ou un alkyle en C₁ à C₄,
Y est un radical nucléophile choisi parmi O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-NH-O, O-C(O)-O-NR₄, NH-C(O)-O-NR₄, C(O)-S, et R₄ est H ou un groupe alkyle en C₁ à C₄,
Z₁, Z₂, Z₃ représentent chacun indépendamment l'un de l'autre, N ou C, de préférence Z₃ représente C, plus préférentiellement Z₃ est C et R₃ est en position Z₃,
R₁ est H, un groupe alkyle en C₁ à C₆, , un alcényle, un groupe aryle, ou un hétérocycle, lesdits groupes alkyle, alcényle, aryle, ou hétérocycle, étant éventuellement substitués par un ou plusieurs groupes fonctionnels choisi parmi les groupes amine, imine, nitrile, cyano, amide, imide, hydroxyle, alcoxyle, carbonyle, carboxyle, ester, thiol, thioéther, thioester et halogénure, de préférence R1 est un groupe méthyle ou éthyle,
R₂ est un groupement protecteur thermolabile, c'est à dire un groupement protecteur qui est stable à température entre 15°C et 25°C, et qui est clivé, dissocié ou relargué d'une molécule à laquelle il est lié, par un traitement thermique à une température comprise entre 50 et 100°C ; et/ou R₂ est un groupement protecteuracidolabile, c'est-à-dire un groupement protecteur qui est stable en condition neutre ou basique et qui est clivé, dissocié ou relargué, par traitement à un pH inférieur à 6,0,
R₃ est H, un groupe alkyle en C₁ à C₁₂, par exemple un groupe iso-propyl, isobutyl, sec-butyl, tert-butyl, isopentyl ou 2,2-diméthylpropyl, un groupe aryle, un hétérocycle, un groupe acyle, un groupe alcényle, un halogène, ou un groupe cyano, lesdits groupes alkyle, , aryle, hétérocycle, ou alcényle, étant éventuellement substitués par un ou plusieurs groupes fonctionnels choisi parmi les groupes amine, imine, nitrile, cyano, amide, imide, hydroxyle, alcoxyle, carbonyle, carboxyle, ester, thiol, thioéther, thioester et halogénure,
et lorsque Y est choisi parmi O, S et NR₄, X n'est pas une liaison.

11. Molécule biologique protégée selon la revendication 10, **caractérisée en ce que** Biomol est choisi parmi les enzymes.

12. Molécule biologique protégée selon la revendication 11 **caractérisée en ce que** Biomol est une enzyme destinée à être utilisée dans une réaction de polymérisation d'acide nucléique, par exemple une ADN polymérase.

13. Utilisation d'un composé selon l'une des revendications 1 à 7 pour l'inactivation réversible d'une enzyme.

14. Utilisation selon la revendication 13, **caractérisée en ce que** l'enzyme est une enzyme destinée à être utilisée dans une réaction de polymérisation d'acide nucléique, par exemple une ADN polymérase.

15. Utilisation d'une molécule biologique protégée selon la revendication 12, dans une réaction d'amplification d'acide nucléique, par exemple pour des applications de démarrage à chaud « Hot Start ».

16. Procédé de déprotection des groupes nucléophiles d'une molécule biologique protégée selon l'une des revendications 9 à 12, ledit procédé comprenant une étape de clivage du ou des groupements thermolabiles et/ou acidolabiles R₂, par traitement thermique et/ou acide respectivement, et la déprotection concomitante des groupes nucléophiles.

17. Procédé de polymérisation d'un acide nucléique comprenant (i) la mise en œuvre d'une molécule biologique protégée selon la revendication 12, (ii) au moins une étape pour la déprotection de ladite molécule biologique, par exemple par un traitement thermique à une température permettant le clivage du ou des groupements thermolabiles R₂, (iii) une étape de polymérisation d'acide nucléique à l'aide de la polymérase déprotégée à l'étape (ii).

## Patentansprüche

1. Eine Verbindung der folgenden Formel (I): 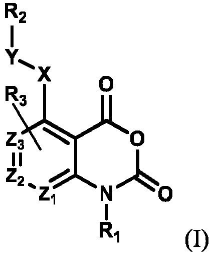 wobei
X ist eine kovalente Bindung oder C₁-C₄-Alkyl,
Y ist ein nucleophiler Rest, ausgewählt aus O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-O-NR₄, NH-C(O)-O-NR₄, C(O)-S, und R₄ ist H oder C₁-C₄-Alkyl,
Z₁, Z₂, Z₃ jeweils unabhängig voneinander N oder C darstellen, vorzugsweise Z₃ C ist, noch bevorzugter Z₃ C ist und R₃ in der Z₃-Position ist,
R₁ H, C₁-C₆-Alkyl, Alkenyl, Aryl oder Heterocyclus ist, wobei das Alkyl, Alkenyl, Aryl oder der Heterocyclus gegebenenfalls mit einer oder mehreren funktionellen Gruppen, ausgewählt aus Amin, Imin, Nitril, Cyano, Amid, Imid, Hydroxyl, Alkoxyl, Carbonyl, Carboxyl, Ester, Thiol, Thioether, Thioester und Halogenid, substituiert ist, vorzugsweise ist R₁ Methyl oder Ethyl, R₂ ist eine thermolabile Schutzgruppe ist, d.h. eine Schutzgruppe, die bei einer Temperatur zwischen 15°C und 25°C stabil ist und die durch Wärmebehandlung bei einer Temperatur zwischen 50°C und 100°C von einem Molekül, an das sie gebunden ist, abgespalten, dissoziiert oder freigesetzt wird; und/oder R₂ eine acidolabile Schutzgruppe ist, d.h. eine Schutzgruppe, die in neutralem oder basischem Zustand stabil ist und die durch Behandlung bei einem pH-Wert unter 6,0 abgespalten, dissoziiert oder freigesetzt wird,
R₃ ist H, C₁-C₁₂-Alkyl, z.B. Isopropyl, Isobutyl, sec-Butyltert-Butyl, Isopentyl oder 2,2-Dimethylpropyl, Aryl, Heterozyklus, Acyl, Alkenyl, Halogen (z.B. F, Cl, Br und I) oder eine Cyanogruppe, wobei die Alkyl-, , Aryl-, Heterocyclus- oder Alkenylgruppen gegebenenfalls mit einer oder mehreren funktionellen Gruppen substituiert sind, die aus Amin-, Imin-, Nitril-, Cyano-, Amid-, Imid-, Hydroxyl-, Alkoxyl-, Carbonyl-, Carboxyl-, Ester-, Thiol-, Thioether-, Thioester- und Halogenidgruppen ausgewählt sind,
und wenn Y aus O, S und NR₄ ausgewählt ist, X keine Bindung ist.

2. Die Verbindung nach Anspruch 1 mit der folgenden Formel (II) :

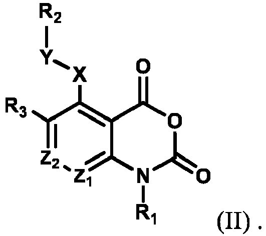

3. Verbindung nach einem der Ansprüche 1 oder 2, **dadurch gekennzeichnet, dass** die thermolabile und/oder acidolabile Gruppe R₂ ausgewählt ist aus *tert-Butoxycarbonyl* (BOC), substituierten oder unsubstituierten Phenoxyacetyl-, Trityl-, Methoxytrityl-, Dimethoxytrityl- oder Citraconylgruppen.

4. Verbindung nach einem der vorangehenden Ansprüche, **dadurch gekennzeichnet, dass** R₁ Methyl ist.

5. Verbindung nach einem der vorangehenden Ansprüche, **dadurch gekennzeichnet, dass** R₃ Iod ist.

6. Verbindung nach einem der vorangehenden Ansprüche, **dadurch gekennzeichnet, daß** Z₁ N und Z₂ C ist.

7. Verbindung nach einem der vorangehenden Ansprüche, **gekennzeichnet durch** eine der folgenden Strukturen:

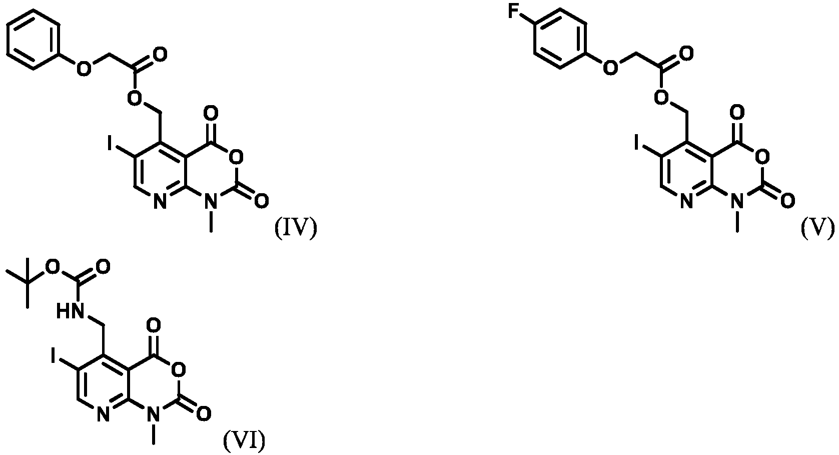

8. Verfahren zur Herstellung eines geschützten biologischen Moleküls, umfassend das Inkontaktbringen einer Verbindung nach einem der Ansprüche 1 bis 7 mit einem biologischen Molekül, das eine oder mehrere nukleophile Gruppen umfasst, unter Bedingungen, die eine Acylierung einer oder mehrerer nukleophiler Gruppen des biologischen Moleküls erlauben, um ein geschütztes biologisches Molekül zu bilden.

9. Geschütztes biologisches Molekül, erhältlich durch das Verfahren nach Anspruch 8.

10. Ein geschütztes biologisches Molekül der folgenden Formel (III) : 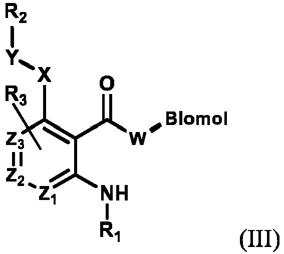 wobei
Biomol ist ein biologisches Molekül
W ist eine nucleophile Gruppe des biologischen Moleküls, z. B. NH, S oder O,
X ist eine kovalente Bindung oder C₁-C₄-Alkyl,
Y ist ein nucleophiler Rest, ausgewählt aus O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-O-NR₄, NH-C(O)-O-NR₄, C(O)-S, und R₄ ist H oder C₁-C₄-Alkyl,
Z₁, Z₂, Z₃ jeweils unabhängig voneinander N oder C darstellen, vorzugsweise Z₃ C ist, noch bevorzugter Z₃ C ist und R₃ in der Z₃-Position ist,
R₁ C₁-C₆-Alkyl, -Alkenyl, -Aryl oder -Heterocyclus ist, wobei das Alkyl, Alkenyl, Aryl oder der Heterocyclus gegebenenfalls mit einer oder mehreren funktionellen Gruppen substituiert ist, die aus Amin-, Imin-, Nitril-, Cyano-, Amid-, Imid-, Hydroxyl-, Alkoxyl-, Carbonyl-, Carboxyl-, Ester-, Thiol-, Thioether-, Thioester- und Halogenidgruppen ausgewählt sind, vorzugsweise ist R₁ Methyl oder Ethyl, R₂ ist eine thermolabile Schutzgruppe, d.h. eine Schutzgruppe, die bei einer Temperatur zwischen 15°C und 25°C stabil ist und die durch Wärmebehandlung bei einer Temperatur zwischen 50 und 100°C von einem Molekül, an das sie gebunden ist, abgespalten, dissoziiert oder freigesetzt wird; und/oder R₂ ist eine acidolabile Schutzgruppe, d.h. eine Schutzgruppe, die in neutralem oder basischem Zustand stabil ist und die durch Behandlung bei einem pH-Wert unter 6,0 abgespalten, dissoziiert oder freigesetzt wird,
R3 H, C₁-C₁₂-Alkyl, z.B. Isopropyl, Isobutyl, sec-Butyl, tert-Butyl, Isopentyl oder 2,2-Dimethylpropyl, Aryl, Heterocyclus, Acyl, Alkenyl, Halogen oder Cyano ist, wobei das Alkyl, , Aryl, Heterocyclus oder Alkenyl, die gegebenenfalls mit einer oder mehreren funktionellen Gruppen, ausgewählt aus Amin-, Imin-, Nitril-, Cyano- , Amid-, Imid-, Hydroxyl-, Alkoxyl-, Carbonyl-, Carboxyl-, Ester-, Thiol-, Thioether-, Thioester- und Halogenidgruppen, substituiert sind,
und wenn Y aus O, S und NR4 ausgewählt ist, X keine Bindung ist.

11. Geschütztes biologisches Molekül nach Anspruch 10, **dadurch gekennzeichnet, dass** Biomol aus Enzymen ausgewählt ist.

12. Geschütztes biologisches Molekül nach Anspruch 11, **dadurch gekennzeichnet, dass** Biomol ein Enzym zur Verwendung in einer Nukleinsäure-Polymerisationsreaktion ist, zum Beispiel eine DNA-Polymerase.

13. Verwendung einer Verbindung nach einem der Ansprüche 1 bis 7 zur reversiblen Inaktivierung eines Enzyms.

14. Verwendung nach Anspruch 13, **dadurch gekennzeichnet, dass** das Enzym ein Enzym zur Verwendung in einer Nukleinsäure-Polymerisationsreaktion ist, beispielsweise eine DNA-Polymerase.

15. Verwendung eines geschützten biologischen Moleküls nach Anspruch 12 in einer Nukleinsäure-Amplifikationsreaktion, zum Beispiel für Heißstart-Anwendungen des Typs "Hot Start".

16. Verfahren zum Entschützen der nukleophilen Gruppen eines biologischen Moleküls, das nach einem der Ansprüche 9 bis 12 geschützt ist, wobei das Verfahren einen Schritt des Abspaltens der thermolabilen und/oder acidolabilen Gruppe(n) R₂ durch Wärme- bzw. Säurebehandlung und des gleichzeitigen Entschützens der nukleophilen Gruppen umfasst.

17. Verfahren zur Polymerisation einer Nukleinsäure, umfassend (i) die Verwendung eines geschützten biologischen Moleküls nach Anspruch 12, (ii) mindestens einen Schritt zur Entschützung des biologischen Moleküls, zum Beispiel durch eine Wärmebehandlung bei einer Temperatur, die die Abspaltung der thermolabilen Gruppe oder Gruppen R₂ erlaubt, (iii) einen Schritt zur Polymerisation der Nukleinsäure unter Verwendung der in Schritt (ii) entschützten Polymerase.

## Claims

1. Compound of the following formula (I): 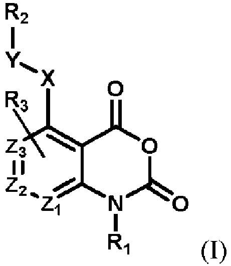 wherein
X is a covalent bond or a C₁-C₄ alkyl,
Y is a nucleophilic group selected from O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-NH-O, O-C(O)-O-NR₄, NH-C(O)-O-NR₄, C(O)-S, and R₄ is H or a C₁-C₄ alkyl group,
Z₁, Z₂, Z₃ each represent, independently of one another, N or C, preferably Z₃ represents C, more preferably Z₃ is C and R₃ is in position Z₃,
R₁ is H, a C₁-C₆ alkyl group, an alkenyl, an aryl group, or a heterocycle, said alkyl, alkenyl, aryl or heterocycle groups being optionally substituted by one or more functional groups selected from the following groups: amine, imine, nitrile, cyano, amide, imide, hydroxyl, alkoxyl, carbonyl, carboxyl, ester, thiol, thioether, thioester, and halide, preferably R₁ being a methyl or ethyl group,
R₂ is a thermolabile protecting group, that is a protecting group which is stable at room temperature between 15°C and 25°C, and which is cleaved, dissociated, or salted out from a molecule to which it is bonded, by thermal treatment at a temperature comprised between 50 and 100°C; and/or R₂ is an acid-labile protecting group, that is a protecting group which is stable in neutral or basic conditions, and which is cleaved, dissociated, or salted out, in acid conditions at room temperature, by treatment at a pH below 6.0,
R₃ is H, a C₁-C₁₂ alkyl group, for example an isopropyl, isobutyl, sec-butyl, tert-butyl, isopentyl, or 2,2-dimethylpropyl group, an aryl group, a heterocycle, an acyl group, an alkenyl group, a halogen (for example F, Cl, Br and I), or a cyano group, said alkyl, aryl, heterocycle or alkenyl groups being optionally substituted by one or more functional groups selected from the following groups: amine, imine, nitrile, cyano, amide, imide, hydroxyl, alkoxyl, carbonyl, carboxyl, ester, thiol, thioether, thioester, and halide,
and when Y is selected from O, S, and NR₄, X is not a bond.

2. Compound according to claim 1, of the following formula (II):

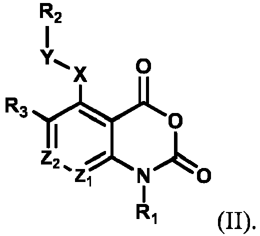

3. Compound according to claim 1 or 2, wherein the thermolabile and/or acid-labile group R₂ is selected from the *t*e*rt*-butoxycarbonyl (BOC), substituted or unsubstituted phenoxyacetyl, trityl, methoxytrityl, dimethoxytrityl, or citraconyl groups.

4. Compound according to any one of the preceding claims, wherein R₁ is a methyl group.

5. Compound according to any one of the preceding claims, wherein R₃ is iodine.

6. Compound according to any one of the preceding claims, wherein Z₁ is N and Z₂ is C.

7. Compound according to any one of the preceding claims, **characterized by** one of the following structures:

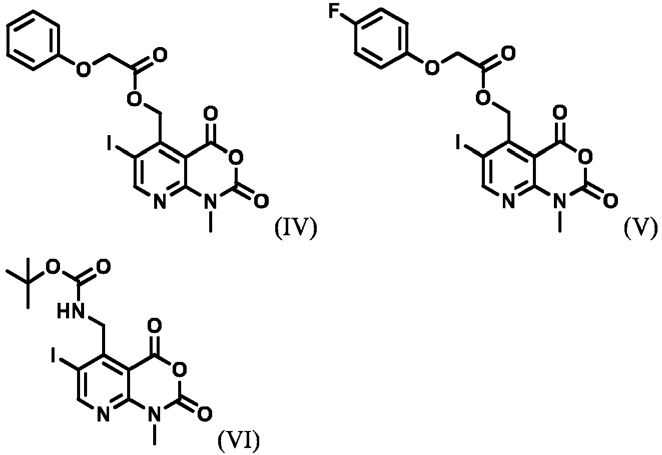

8. Method for preparing a protected biological molecule, comprising the placing of a compound according to one of claims 1 to 7 in contact with a biological molecule comprising one or more nucleophilic groups, under conditions permitting the acylation of one or more nucleophilic groups of said biological molecule in order to form a protected biological molecule.

9. Protected biological molecule, obtainable by the method according to claim 8.

10. Protected biological molecule of the following formula (III): 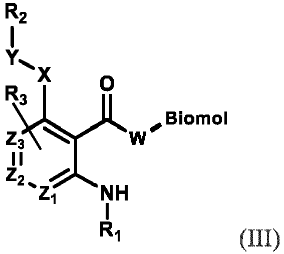 wherein
Biomol is a biological molecule
W is a nucleophilic group of the biological molecule, for example NH, S, or O,
X is a covalent bond or a C₁-C₄ alkyl,
Y is a nucleophilic radical selected from O, S, NR₄, O-NR₄, NH-O, NH-NR₄, C(O)-O-NR₄, C(O)-NH-O, C(O)-NH-NR₄, O-C(O)-NH-NR₄, NH-C(O)-NH-NR₄, O-C(O)-NH-O, NH-C(O)-NH-O, O-C(O)-O-NR₄, NH-C(O)-O-NR₄, C(O)-S, and R₄ is H or a C₁-C₄ alkyl group,
Z₁, Z₂, Z₃ each represent, independently of one another, N or C, preferably Z₃ represents C, more preferably Z₃ is C and R₃ is in position Z₃,
R₁ is H, a C₁-C₆ alkyl group, an alkenyl, an aryl group, or a heterocycle, said alkyl, alkenyl, aryl or heterocycle groups being optionally substituted by one or more functional groups selected from the following groups: amine, imine, nitrile, cyano, amide, imide, hydroxyl, alkoxyl, carbonyl, carboxyl, ester, thiol, thioether, thioester, and halide, preferably R₁ being a methyl or ethyl group,
R₂ is a thermolabile protecting group, that is a protecting group which is stable at room temperature between 15°C and 25°C, and which is cleaved, dissociated, or salted out from a molecule to which it is bonded, by thermal treatment at a temperature comprised between 50 and 100°C; and/or R₂ is an acid-labile protecting group, that is a protecting group which is stable in neutral or basic conditions, and which is cleaved, dissociated, or salted out, in acid conditions at room temperature, by treatment at a pH below 6.0,
R₃ is H, a C₁-C₁₂ alkyl group, for example an isopropyl, isobutyl, sec-butyl, tert-butyl, isopentyl, or 2,2-dimethylpropyl group, an aryl group, a heterocycle, an acyl group, an alkenyl group, a halogen, or a cyano group, said alkyl, aryl, heterocycle or alkenyl groups being optionally substituted by one or more functional groups selected from the following groups: amine, imine, nitrile, cyano, amide, imide, hydroxyl, alkoxyl, carbonyl, carboxyl, ester, thiol, thioether, thioester, and halide,
and when Y is selected from O, S, and NR₄, X is not a bond.

11. Protected biological molecule according to claim 10, wherein Biomol is selected from enzymes.

12. Protected biological molecule according to claim 11, wherein Biomol is an enzyme intended for use in a nucleic acid polymerization reaction, for example a DNA polymerase.

13. Use of a compound according to one of claims 1 to 7 for the reversible inactivation of an enzyme.

14. Use according to claim 13, wherein the enzyme is an enzyme intended for use in a nucleic acid polymerization reaction, for example a DNA polymerase.

15. Use of a protected biological molecule according to claim 12 in a nucleic acid amplification reaction, for example for hot start applications.

16. Method for the deprotection of nucleophilic groups of a protected biological molecule according to one of claims 9 to 12, said method comprising a step of cleaving the thermolabile and/or acid-labile group or groups R₂, respectively by heat and/or acid treatment, and concomitant deprotection of the nucleophilic groups.

17. Method for the polymerization of a nucleic acid, comprising (i) the use of a biological molecule protected according to claim 12, (ii) at least one step for the deprotection of said biological molecule, for example by heat treatment at a temperature allowing cleavage of the thermolabile group or groups R₂, (iii) a nucleic acid polymerization step using the polymerase deprotected in step (ii).
